# EUROPEAN PATENT APPLICATION

(11) **EP 1 586 330 A1**
(43) Date of publication of application: **19.10.2005**
(21) Application number: 04009135.7
(22) Date of filing: 16.04.2004
(51) Int. Cl.: A61K 39/02, A61K 39/12, A61K 39/002

(54) **Vaccination against malignant melanoma**

(71) Applicant: Georg-August-Universität Göttingen, 37075 Göttingen (DE)
(72) Inventor: Krone, Bernd, 37073 Goettingen (DE); Hunsmann, Gerhard, 37077 Goettingen (DE)
(74) Representative: Wichmann, Hendrik

(57) **Abstract**

The present invention relates to a vaccination which reduces the risk to contract melanoma to about half to third and moreover, within a five year period post diagnosis of malignant melanoma the risk to die is reduced to about half as compared to the non-vaccinated individuals.

Beyond its effect on melanoma, the vaccination of the invention also may have protective effects against some other malignancies such as cancers of bladder, breast, esophagus, and kidney, against sarcomas and lymphomas, and against a multitude of micro-organisms which can induce severe and lethal infections or allergies.

The present invention relates also to the use the yellow fever vaccine for the prophylactic vaccination against a malignancy, in particular melanoma, to the use of some other licensed vaccinations for the prophylactic vaccination against a malignancy, in particular melanoma, and to new synthetic vaccines which use the identified antigenic motifs.

## Description

The vaccinations of the invention reduce the risk to contract melanoma to about half to third as compared to the non-vaccinated individuals. However, if melanoma develops, the vaccinations reduce also the risk to die from malignant melanoma within a five year post diagnosis period to about half as compared to the non-vaccinated individuals.

Beyond its effect on melanoma, the vaccination of the invention also may have protective effects against some other malignancies such as cancers of bladder, breast, esophagus, and kidney, against sarcomas and lymphomas, and against a multitude of micro-organisms which can induce severe and lethal infections or allergies.

The present invention relates also to the use of the yellow fever vaccine for the prophylactic vaccination against a malignancy, in particular melanoma. As alternatives some other licensed vaccinations which today are also given only for special indications not including tumor protection are provided for the prophylactic vaccination against a malignancy, in particular melanoma. Furthermore, the invention relates to new synthetic vaccines which use the identified antigenic motifs (Table 1). Moreover, it is shown that both of the old vaccines (classical vaccinia and BCG) produce a synergistic concomitant melanoma risk reduction in patients with a skin type Fitzpatrick I (white, always reacting with sunburn) and in patients with multiple episodes of sunburns. Thus, the vaccinations of the invention will be most useful in persons who are already the main target group of melanoma information and prevention campaigns. However, the vaccination(s) will be suitable for application to the general population.

### BACKGROUND OF THE INVENTION

Melanoma is a malignant tumor of the skin. It contributes about 2-4% to all human malignancies. The prevalence of melanoma is increasing. There is a surgical cure for melanoma in its early stage. However, despite some controversies no established adjuvant therapy exists to reduce the risk of a relapse. Cancers of bladder, breast, esophagus, and kidney, and sarcomas, and lymphomas contribute about 3%, 8%, 2%, 3%, 1%, and 5% to all malignancies, respectively.

In addition, infectious diseases remain major causes of death and disease. High standards of hygiene and antibiotic therapy have led to a reduced para-specific immunity against many micro-organisms. Some organisms can induce severe and lethal infections. Other micro-organisms, however, do not even cause disease or cause only mild disease. In developed countries humans are only rarely infected with micro-organisms which do not cause disease or cause only mild disease but which seemingly also induce protection against other organisms that can cause severe and lethal infections or allergies (para-specific immunity). Thus, para-specific immunity is not wide-spread in developed countries.

Vaccinations with vaccinia, BCG, and measles vaccine have been described to correlate with a good non-specific immunity against infectious diseases and with overall survival of immunized versus un-immunized siblings. The immunological basis of these observations has yet to be established (1, 2).

Classical vaccinia vaccination against smallpox and BCG vaccination against tuberculosis were tried to *cure* malignant diseases including melanoma. Beneficial effects were seen in individual cases, however, vaccinia and BCG vaccinations neither proved useful for cure nor for a general or adjuvant therapy of a malignant disease (3).

Exceptionally, as an adjuvant therapy of bladder carcinoma, repeated instillation of the BCG vaccine into the bladder is an established treatment (4). More then a century ago, Coley and followers used antigens from *Serratia marcescens* and *Streptococcus pyogenes* in an attempt to cure sarcomas, melanomas and some carcinomas (5). A number of possible targets for melanoma therapy have been identified. In the frame of works to identify possible targets for *therapeutic* vaccinations, Schiavetti et al. discovered the HERV K-MEL antigen (6).

There are several reports, mainly epidemiological studies, which state that the BCG vaccination reduces the risk of childhood leukemia and of some other malignant diseases (7). However, other studies could not confirm these reports. Also infectious diseases are reported to correlate somehow with a reduced risk of malignancies (8). Only recently studies on malignant melanoma showed that classical vaccinations against smallpox had the same melanoma protective effect as did BCG vaccination and, most importantly, that the strong risk lowering effects of the two vaccinations, classical vaccinia and BCG, were not cumulative (9, 10). In summary, because of conflicting results the effect of BCG vaccination on tumor protection was not clear.

There are indications that classical smallpox vaccination had also a positive influence on the general status of health of the population, although this could not be proven (1). Vaccines such as BCG and measles are reported to have non-specific positive effects on the survival within the first year of life in an area where many children die from infectious diseases within their first year of life (2).

Vaccinations given against smallpox and/or tuberculosis in early childhood reduce(d) the melanoma risk in all age groups to 1/3, whereas in persons below 50 years of age the melanoma risk was lowered to 1/6 (9, 10, 11, 12). Both vaccinations are not used any more, or rather, BCG is in use only in some mostly non-developed countries. The vaccinations against smallpox were abandoned about 28 years ago. About 13 years ago the vaccinations against tuberculosis were considered obsolete in most developed countries. Earlier the coverage of the population in Europe with the two vaccinations was 90% and 50%, respectively. Thus, there is a need to provide vaccination(s) with an effect similar to classical vaccinia or BCG vaccination for the purpose of melanoma protection to all newborns and to all persons who are vaccinated neither against smallpox nor against tuberculosis. There is also a need to boost the protection by repeated vaccination later in life in all persons who received one or both of the above-mentioned old vaccinations in early childhood, because the protective effect of the vaccination(s) is waning with time.

In addition, para-specific immunity resulting from previous infections with other micro-organism(s) is induced less effectively in developed countries due to high standards of hygiene and antibiotic therapies. There is also a need for vaccination(s) providing a para-specific immunity and giving some protection against many infections causing severe and lethal infectious diseases or allergies.

### SUMMARY OF THE INVENTION

The present inventors were faced with the question why vaccinia and BCG vaccination(s) were melanoma protective. Unexpectedly, the present inventors have found that the two vaccines as well as >70 human pathogenic micro-organisms comprise polypeptides having significant homologies to the target antigen on melanoma cells and on their presumed precursors, the congenital and dysplastic naevi cells - the peptide HERV K-MEL. The present inventors list in Table 2 the typical causative agents of the diseases. All these causative agents had significant sequence homology with SEQ ID NO:1. This homology further explains the presentation of this class of target antigens to the immune system through the HLA A2 class-I antigen (6). The antigens with sequence homology to HERV K-MEL have the anchor amino acids for presentation with HLA A2 class-I antigen as does the target antigen HERV K-MEL. Thus, based on the insight gained by the present invention, they all fulfill the theoretical requirements for immune presentation. The present inventors further identified existing vaccines, which had polypeptides with homology to the target antigen including the above-mentioned anchor sequences. Among them are licensed vaccines, and, based on the insight gained by the present invention, as the best candidate the yellow fever vaccine is considered. Alternatives are the following life vaccines: the vaccines against Japanese encephalitis B virus, Venezuelan equine encephalitis virus, the oral vaccines against cholera and typhoid fever, a life vaccine against rickettsia typhi and, in addition, the Friedmann vaccine *(Mycobacterium chelonae).* Alternatives are the following inactivated vaccines against pertussis (whole cell vaccine), anthrax, Q-fever, respiratory syncytial virus, pneumococci, and plague. Further alternatives are the experimental vaccine against Lyme borreliosis and a vaccine against brucellosis, which is only licensed in veterinary medicine. Furthermore, Coley's toxin is a candidate vaccine which is not yet licensed, but should also be explored for the purpose of tumor prophylaxis. In all these vaccines the motif which is predicted to induce cross-reactive immune responses against the HERV K-MEL tumor antigen was identified.

By the vaccination(s) of the invention also the risk to die within a five year post diagnosis period from malignant melanoma will be reduced to about half as compared to the non-vaccinated individuals (12).

Beyond its effect on melanoma, based on the insight gained by the present invention, the vaccination(s) are predicted to have also protective effects against some other malignancies such as cancers of bladder, breast, esophagus, and kidney, against sarcomas and lymphomas, and against a multitude of micro-organisms which can induce severe and lethal infections or allergies.

In addition, based on the insight gained by the present invention, the vaccination(s) will provide a para-specific protection (1), against some other malignancies and (2), against (at least some) very severe and often lethal infectious diseases or allergies where the causative agent has also the homologue to HERV K-MEL homology. 1.) The HERV K-MEL antigen is presented partly in some other malignancies, for example cancers of the bladder [in a frequency of 8/19], breast [10/21], and esophagus [2/10], and sarcomas [9/21 cases] (6), and was also found in kidney cancer and lymphoma. Based on the insight gained by the present invention, the antigen-positive malignant entities should also be susceptible to a prophylaxis by means of the proposed vaccination(s). 2.) The course of at least some of these often dangerous infections will be modified. It should be noticed that expositions to these infections lead sometimes to severe and lethal diseases, but quite often are abortive or sub-clinical infections. Humans are protected by diverse non-specific defense mechanisms. One of these defense mechanisms is a good para-specific immune response. In the developed countries this kind of response is today rarely induced by previous expositions to other micro-organisms, which thereby induce also cross-reactive immune reactions. The HERV K-MEL homology, because of its abundance (and because of a presumed physiological function) is an important target. The vaccine(s) proposed by the present inventors will induce cross-reactive immune responses and will reduce the number of severe infectious diseases and of allergies of different origin.

In its most preferred embodiment, the invention provides an already licensed vaccination which has proved to be harmless on the basis of more than 200 million persons vaccinated and on the basis of more than 50 years of experience, the yellow fever vaccine, for the prophylaxis of malignancies, in particular for the prophylaxis of melanoma. This vaccination is at present not given to the general population but only to persons who travel into regions of the world where yellow fever is endemic, parts of Africa and South America. However, the inhabitants of these regions are only rarely vaccinated because these countries are poor. As alternative embodiments the invention provides some other licensed vaccinations which today are also given only for special indications not including tumor protection. The invention further relates to new synthetic vaccines which use the identified antigenic motifs (Table 1). Moreover, the inventors could show that both of the old vaccines (classical vaccinia and BCG) produce a synergistic concomitant melanoma risk reduction in patients with a skin type Fitzpatrick I (white, always reacting with sunburn) and in patients with multiple episodes of sunburns. Thus, the vaccines of the invention will be most useful in persons who are already the main target group of melanoma information and prevention campaigns. However, the vaccination(s) will be most effective when applied to the general population.

### DEFINITIONS

A "polypeptide" as used herein is a molecule comprising more than 20, in particular more than 25, 30, 40, 50, 60 and most preferably more than 70 amino acids, but less than 10000, in particular less than 9000, 8000, 7000, 6000, 5000, 4000, 3000 or 2000, most preferably less than 1500 amino acids. Also, polypeptides with substantial amino acid sequence identity and polypeptides, which contain a low percentage, e.g. less than 5%, 3% or even only up to 1%, of modified or non-natural amino acids are encompassed.

A "peptide" as used herein is a molecule comprising less than 20 amino acids, preferably less than 18, 16 or 14 amino acids, but preferably more than 4, 5, 6, 7, 8 or 9 amino acids.

A "not naturally occurring polypeptide" is a polypeptide, which is not encoded as such by the genome of a naturally occurring species, in particular a polypeptide that is not identical to one of those polypeptides of the Genbank database as of March 15, 2004, with a naturally occurring species identified as their source. This means that a " not naturally occurring polypeptide " polypeptide does not occur as such in nature, but can be, and in particular was, produced by laboratory manipulations, such as genetic engineering techniques or chemical coupling of another molecule to a polypeptide. This term in particular encompasses all mutant polypeptides, in particular deletions, truncations, multiple substitutions and fusion polypeptides, which at one stage were produced by genetic engineering techniques. The term "not naturally occurring polypeptide " polypeptide as used herein preferably does not include fusion polypeptides with a component (b), which is a "tag" commonly used for facilitated protein purification, like a multi-histidine-tag or a GST-tag, or which is a polypeptide commonly used in so-called two-hybrid assays, like a Gal4-domain.

The term "at least one", as used here, means "one and more than one", particularly one, two, three, four and five.

The term "at least X% identity with Y" as used herein means that if a given nucleic acid or polypeptide sequence is aligned to a nucleic acid sequence Y or a polypeptide sequence Y, respectively, X% of the bases or amino acids within the aligned region are identical. X can be at least 70, at least 75, at least 80, at least 85, at least 90 or at least 95. If polypeptide sequences are aligned, then the closely related residues aspartic acid and glutamic acid are usually regarded as being identical, as well as the closely related residues asparagine and glutamine. However, preferably the residues asparagine and glutamine are not regarded as being identical and more preferably also the residues aspartic acid and glutamic acid are also not regarded as being identical.

A "causative agent" as used herein is an agent leading to a disease via its presence and in particular via its proliferation in an infected host, for example a bacterium leading to a bacterial infection in a human being or a virus leading to a viral infection in a human being or a protozoan leading to a protozoan infection in a human being or a fungus leading to a fungal infection in a human being.

A "defined homology with HERV-K-MEL" is used herein as
I) meaning that a peptide with defined homology has at least 5, more preferably 6 and most preferably 7, amino acid residues in common with SEQ ID NO:1 when aligned with SEQ ID NO:1 to maximize the number of identical residues in such a way that only at position 5 and position 6 an insertion or deletion of one amino acid residue is allowed, or alternatively
II) meaning that a peptide with defined homology is derived from the peptide sequence MLAVISCAV or MLAVVSCAV in such a way that a) either residue M at position 1 and residue A at position 8 remain unchanged, or that b) residue L at position 2 and residue V at position 9 remain unchanged; and that one, two or three, more preferably one or two, even more preferably one, of the other amino acid residues are exchanged for another amino acid residue, preferably wherein the amino acid exchange is a semi-conservative exchange, more preferably wherein the amino acid exchange is a conservative exchange, and/or wherein one or two amino acid residues can be inserted or deleted at any position between amino acid residue 1 and 8 in the case of alternative a) or amino acid residue 2 and 9 in the case of alternative b); wherein preferably said insertion is an insertion of one or two, more preferably one, amino acid residues at position 5 or 6, or wherein preferably said deletion is a deletion of one or two, more preferably one, amino acid residues at position 5 or 6.

"Aligned to maximize the number of identical residues" as used herein means that particular alignment of two peptides with one another wherein the highest number of identical amino acids are at matching positions. If no single alignment gives a higher number of identical amino acids at the matching positions than all the other alignments, than those n alignments which all give the same highest number of identical amino acids at matching positions are used for determining whether an aligned peptide has a defined homology with HERV-K-MEL.

"Amino acid" as used herein is any one of the alpha-amino acids that are the chief components of proteins, in particular any one of the 21 alpha-amino acids alanine, valine, leucine, isoleucine, proline, methionine, phenylalanine, tyrosine, tryptophan, histidine, glycine, serine, threonine, aspartic acid, asparagine, glutamic acid, glutamine, lysine, arginine, cysteine and selenocystein, and more particularly any one of the 20 amino acids alanine, valine, leucine, isoleucine, proline, methionine, phenylalanine, tyrosine, tryptophan, histidine, glycine, serine, threonine, aspartic acid, asparagine, glutamic acid, glutamine, lysine, arginine and cysteine.

The expression "semi-conservative amino acid exchange" and the expression "conservative amino acid exchange" as used herein are explained by the following table

| Amino acid | Conservative amino acid exchange is to | Semi-conservative amino acid exchange includes additionally an exchange to |
|---|---|---|
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y; F; K; R | L; M; A |
| I | V;L;M;A | F;Y;W;G |
| K | R; H | D; E; N; Q; S; T; A |
| L | M;I;V;A | F;Y;W;H;C |
| M | L; I; V; A | F; Y; W; C; |
| N | Q | D; E; S; T; A; G; K; R |
| P | V;I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F; Y; H | L;M;I;V;C |
| Y | F; W; H | L; M; I; V; C |

Changing from A, F, H, I, L, M, P, V, W or Y to C is semi-conservative if the new cysteine remains as a free thiol. Furthermore, the skilled person will appreciate that a glycine at a sterically demanding position should not be substituted and that P should not be introduced into parts of the protein which have an alpha-helical or a beta-sheet structure.

"Prevention" as used herein is independent of any immunologic theory how it is achieved. A vaccination is considered effective when the risk to contract the envisaged disease or to die from this disease, for example melanoma, is significantly reduced.

"Anchor sequence" as used herein are the residues of a peptide necessary for its presentation by a, preferably human, MHC complex, for example MHC I or MHC II, but preferably MHC I. These are, for the example of the HLA-A2, a residue M at position 1 and a residue A at position 8 of a peptide to be presented, or a residue L at position 2 and a residue V at position 9 of a peptide to be presented. Peptides eluted from a given HLA-molecule share the anchor residues. Often the second or fifth and the eight or ninth positions are shared by all the peptides binding to a given HLA-molecule. However these anchor patterns are unique for each MHC type. Although the anchor residues are conserved, the remainder of the peptide sequence can vary widely.

"HLA" as used herein is the abbreviation for Human Leukocyte Antigen. Within the meaning of the present invention HLA and the term Major Histocompatibility Complex (MHC) are used interchangeably and refer to the actual antigen-presenting protein complex on the cell surface and not to the genes encoding said complex. Humans have a set of different proteins of the Major Histocompatibility Complex (MHC). Each person inherits the ability to express two copies of HLA-A, -B, and -C MHC class I proteins. Thus, the tissue type includes up to six different class I alleles.

"Adjuvant" as used herein is a substance increasing the immunogenic effect of an antigen. The possible modes of action of an adjuvant are a) the formation of a depot of antigen primarily at the site of application and from which the antigen is released, b) the increased uptake of antigen into antigen presenting cells, particularly dendritic cells (DC), macrophages, and B lymphocytes, c) the induction of synthesis and secretion of enhancing factors, such as cytokines, which act principally but not exclusively on cells of the immune system, especially T and B lymphocytes. A wide range of compounds possess adjuvant properties: aluminum compounds, oil emulsions, detoxified lipopolysaccharides, peptides, liposomes, purified saponin, and polymeric species.

The term "pharmaceutically acceptable carrier" as used herein refers to substances and compositions that do not produce an adverse, allergic or other untoward reaction when administered to an animal or a human in particular. As used herein, a "pharmaceutically acceptable carrier" includes all inert, non-toxic, liquid or solid fillers or diluents, as long as they do not react with the polypeptide of the invention in an inappropriate negative manner, solvents, dispersion media, coatings, antibacterial and antifungal agents, isotonic and absorption delaying agents, and the like, for example liquid pharmaceutical carriers like sterile water, saline, sugar solutions, ethanol and/or certain oils. Carriers for the production of capsules and tablets may include binders and fillers.

"Unit dosage" as used herein is a single dose that is the dosage of a medicament used for one administration.

### DETAILED DESCRIPTION OF THE PREFERRED EMBODIMENTS

For several years scientists could not find an approach to identify the immunological basis of the protection by Coley's toxin therapy. The present inventors have identified the target antigen and have further found that there are sequence homologies of this target antigen with polypeptides of further vaccines and bacteria. The present inventors found experimental proof for the concept of para-immunity induced by the vaccines of the invention.

The immune response of four rhesus monkeys vaccinated with yellow fever vaccine has been investigated. Before vaccination, sera of the four monkeys showed no immune reaction against two melanoma cell lines (titre 1: < 10). Four weeks after vaccination with the yellow fever vaccine, sera from these animals showed reactions against the two melanoma lines with titres around 1:160 (for details see Table 3, for experimental details see section "examples"), which demonstrated the ability of the inventive vaccines to induce a cross-reactive immune response against the causative agent of another disease than yellow fever, namely melanoma cells.

Vaccines of the invention can thus be used to induce a para-specific or non-specific immunity against infectious diseases. The micro-organisms present in vaccines of the invention do not only induce a cross-reactive immune response (antibody and perhaps CTC responses) against the human HERV K-MEL but also to some extent cross-reactive immune responses against each other on the basis of the widely distributed sequence homology found by the inventors. Therefore, a vaccine which protects against a tumor, based on the inventive concept of the discovered HERV K-MEL homology, will, based on the insight gained by the present invention, also provide some protection against an infectious disease caused by another micro-organism with a HERV K-MEL homology (examples see Table 1).

The invention therefore relates to the use of a first peptide with defined homology to SEQ ID NO:1 (HERV K-MEL) and/or a composition or a polypeptide comprising said first peptide sequence for the preparation of a vaccine for the prevention of a disease, wherein one of the causative agents of said disease comprises a polypeptide comprising a second peptide sequence with defined homology to SEQ ID NO:1 (HERV K-MEL) which second peptide sequence is not identical with said first peptide sequence, wherein the vaccine is not a vaccinia vaccine or the BCG vaccine. Thus, the first peptide with defined homology to SEQ ID NO:1 (HERV K-MEL) is used to generate a para-immune response against a causative agent of another disease, in which the causative agent comprising a polypeptide with the first peptide sequence is not involved, the causative agent of said other disease comprising a polypeptide with said second peptide sequence with defined homology to SEQ ID NO:1 (HERV K-MEL). To use an example, the yellow fever vaccine, comprising a polypeptide with a first peptide sequence with defined homology to SEQ ID NO:1 (HERV K-MEL), namely the peptide LAVSSAV (SEQ ID NO:94), when used in accordance with this invention, is not used for the prevention of yellow fever, but for the prevention of the other diseases mentioned herein, the causative agents of which all comprise a polypeptide with a different second peptide sequence with defined homology to SEQ ID NO:1 (HERV K-MEL), which second peptide sequence is not identical with the mentioned first peptide sequence of the yellow fever vaccine, for example the use of the yellow fever vaccine for the prevention of melanoma. Thus, in general, a vaccine against disease X, when used in accordance with this invention, is used for the prevention of the other diseases mentioned, for example for the prevention of melanoma, but not for the prevention of disease X.

Preferably, the disease is a malignancy, and more preferably said malignancy is selected from the group consisting of melanoma, carcinoma of bladder, carcinoma of kidney, carcinoma of esophagus, sarcoma, lymphoma and leukemia, more preferably said malignancy is melanoma.

Immune surveillance of melanoma and of some other malignancies seems to be a feature of an immune defense mechanism which depends on previous expositions to external stimuli. The present inventors identified that the causative agents of "melanoma-protective diseases" as well as the two vaccines have polypeptides with a defined sequence homology with the HERV K-MEL antigen (6). This antigen is a nona- or dekapeptide coded by an endogenous human retrovirus. For example, with the HLA-A2 class-I molecules said antigen presents itself to the immune system without proteolytic processing. The antigenic peptide was shown earlier not to be expressed in normal body cells but to be presented on most melanomas and in most persons also on dysplastic and congenital naevi - potential melanoma precursors (6). The homologous sequences to said antigen identified by the present inventors in 75 pathogenic micro-organisms of humans include also the anchors needed for presentation with the HLA-A2 molecule. The immune surveillance can thus be induced by prophylactic vaccination and maintained over decades. It is predicted, without wishing to be bound by any theory, that upon emergence of said tumour antigen HERV-K-MEL, which correlates with the risk of malignancy, the immune surveillance recognizes those cells which could result in malignant transformation years or even decades later. These cells can be destroyed or hindered to proceed on their way to malignancy.

In a preferred embodiment, said disease is an infectious disease, and preferably said infectious disease is selected from the group consisting of acute respiratory disease, African sleeping disease, anthrax, arthritis, babesiosis, chagas disease, chancroid, cholera, dell wart, diphtheria, duodenal ulcera, ecthyma contagiosum, endemic typhus, enteritis, epidemic typhus, erysipelas, gastritis, gonorrhea, histoplasmosis, intra-abdominal and pelvic infections, Japanese encephalitis B, kala-azar, leprosy, listeriosis, local invasive infection, lyme disease, lymphogranuloma inguinale, malaria tropica, meningitis, morbus Bang, morbus Weil, Oroya fever, peritonitis, pertussis, plague, pneumonia, Q fever, rocky mountain spotted fever, sepsis, sinusitis, smallpox, swimming pool granuloma, syphilis, tetanus, tick-borne encephalitis, toxoplasmosis, trachoma, trichomoniasis, tuberculosis, ulcera, urinary tract infection, Venezuelan equine encephalitis, wound infection, and yellow fever, more preferably said disease is selected from the group consisting of acute respiratory disease, anthrax, arthritis, chancroid, enteritis, histoplasmosis, intra-abdominal and pelvic infections, leprosy, local invasive infection, lyme disease, lymphogranuloma inguinale, morbus Bang, peritonitis, sinusitis, toxoplasmosis, and trichomioniasis, and most preferably said disease is selected from the group consisting of African sleeping disease, chagas disease, cholera, gonorrhea, kala-azar, listeriosis, malaria tropica, meningitis, morbus Weil, pneumonia, sepsis, syphilis, trachoma, tuberculosis, urinary tract infection, and wound infection.

The above-mentioned diseases are caused by micro-organisms which have polypeptides which comprise a peptide sequence with defined sequence homology to HERV-K-MEL, and these micro organisms might also be controlled by the immune surveillance which can be induced by prophylactic vaccination(s) of the invention. The vaccination(s) make use of cross-reacting immune responses involving the homology to HERV-K-MEL. The importance of this phenomenon results from the circumstance that micro-organisms which are frequent or rare but which can cause very severe infectious diseases have polypeptides with the sequence homology to HERV-K-MEL. For example a dirty dozen of micro-organisms or toxins is defined which are regarded the main candidates for bio-terrorism. Eight of these candidates for bio-terrorism-micro-organisms have said sequence homology to HERV-K-MEL.

In a preferred embodiment said first peptide with defined homology and/or said second peptide with defined homology each have at least 5, more preferably 6 and most preferably 7, amino acid residues in common with SEQ ID NO:1 when aligned with SEQ ID NO:1 to maximize the number of identical residues in such a way that only at position 5 and position 6 an insertion or deletion of one amino acid residue is allowed. For example, one or two, preferably only one, amino acid(s) may be inserted between amino acids corresponding to amino acids V and I of the nonapeptide MLAVISCAV or between amino acids corresponding to amino acids I and S of said nonapeptide, or an amino acid may be inserted between amino acids corresponding to amino acids V and I of the decapeptide AMLAVISCAV or between amino acids corresponding to amino acids I and S of said decapeptide.

In a further preferred embodiment, said first peptide with defined homology and/or said second peptide with defined homology each are derived from the peptide sequence MLAVISCAV or MLAVVSCAV in such a way that a) either residue M at position 1 and residue A at position 8 remain unchanged, or that b) residue L at position 2 and residue V at position 9 remain unchanged,
and that one, two or three, more preferably one or two, even more preferably one, of the other amino acid residues are exchanged for another amino acid residue, preferably wherein the amino acid exchange is a semi-conservative exchange, more preferably wherein the amino acid exchange is a conservative exchange, and/or wherein one or two amino acid residues can be inserted or deleted at any position between amino acid residue 1 and 8 in the case of alternative a) or amino acid residue 2 and 9 in the case of alternative b); wherein preferably said insertion is an insertion of one or two, more preferably only one, amino acid residues at position 5 or 6, or wherein preferably said deletion is a deletion of one or two, more preferably only one, amino acid residues at position 5 or 6. For example, one or two, preferably only one, amino acid(s) may be inserted between the amino acids V and I of the nonapeptide MLAVISCAV or between the amino acids I and S of said nonapeptide, or between the amino acids V and V of the nonapeptide MLAVVSCAV or between the amino acids V and S of said nonapeptide.

In a further preferred embodiment the first and/or second peptide is selected from the group consisting of SEQ ID NO:1-97, preferably selected from any one from the following groups consisting of SEQ ID NO: 3-13 or SEQ ID NO: 14-24 or SEQ ID NO: 25-35 or SEQ ID NO: 36-46 or SEQ ID NO: 47-57 or SEQ ID NO: 58-67 or SEQ ID NO: 68-77 or SEQ ID NO: 78-87 or SEQ ID NO: 88-97. SEQ ID NO:3-97 are examples of peptides with defined homology to HERV-K-MEL which were found by a database search in polypeptides of some 75 pathogenic micro-organisms of humans.

In a further preferred embodiment said first peptide with defined homology to SEQ ID NO: 1 (HERV K-MEL) and/or said composition comprising said first peptide sequence is comprised in a vaccine and said vaccine is selected from the group consisting of the yellow fever vaccine, the Japanese encephalitis B vaccine, the Venezuelan equine encephalitis vaccine, the typhoid fever vaccine, the cholera vaccine, the rickettsia typhi vaccine, the pneumococci vaccine, the Friedmann vaccine (Mycobacterium chelonae), the vaccine against respiratory syncytial virus, the vaccine against pertussis, the vaccine against tick borne encephalitis, the Coley's toxin vaccine, the vaccine against Q-fever, the vaccine against brucella, the vaccine against anthrax, the vaccine against plague and the modified vaccinia Ankara (MVA), preferably selected from the group consisting of the yellow fever vaccine, the Japanese encephalitis B vaccine, the Venezuelan equine encephalitis vaccine, the typhoid fever vaccine, the cholera vaccine and the rickettsia typhi vaccine, and most preferably the yellow fever vaccine. These vaccines will provide a subject with the desired melanoma protection and furthermore with protection against many infections causing severe and lethal infectious diseases or allergies. Intranasal administration of the yellow fever vaccine is particularly preferred.

The composition of said vaccines is well known to a person skilled in the art. The mode of preparation and/or composition of such registered vaccines can, for example, be determined by sending a request to the German Paul-Ehrlich-Institute, Bundesamt für Sera und Impfstoffe, Langen, which is keeping records of registered vaccines. In the United States the corresponding Official Institute is the United States Food and Drug Administration.

It is further preferred that the vaccine is to be administered to a human subject above age 10, in those case wherein the human subject has previously been vaccinated as a child with an above-mentioned vaccine or with smallpox- or BCG-vaccine. This is done to boost the already existing, but waning immune response against HERV-K-MEL in those subjects, i.e. in subjects which had been vaccinated as a child with the vaccinia or BCG vaccine.

In a further preferred embodiment, the vaccine is to be administered to a child below age 2 years, more preferably to a baby below age 1 year. This is done to provide vaccination(s) with an effect similar to classical vaccinia or BCG vaccination for the purpose of melanoma protection to all newborns. Furthermore the vaccination(s) provides a para-specific immunity and gives some protection against many infections causing severe and lethal infectious diseases or allergies. But also all persons who are vaccinated neither against smallpox nor against tuberculosis and are therefore likely to have no protective immunity against melanoma may benefit from the vaccination of the invention.

It is preferred that the vaccine has further been adapted so as to be administrable to a child below age 2 years, preferably to a baby below age 1 year. This is achievable by lowering the volume to be administered to the child or baby in comparison to the dosage which would be administered to an adult human being, and by using diluents and adjuvants considered safe for use in small children, and will avoid or reduce the risk of an untoward reaction of the child or baby to the vaccine.

The invention further relates to a method of vaccinating a subject for the prophylaxis of a disease as specified above, preferably wherein the disease is a malignancy, more preferably wherein the malignancy is melanoma, wherein the method comprises the step of administering a first peptide as defined above, preferably wherein said first peptide has at least 5, more preferably 6 and most preferably 7, amino acid residues in common with SEQ ID NO:1 when aligned with SEQ ID NO:1 to maximize the number of identical residues in such a way that only at position 5 and position 6 an insertion or deletion of one amino acid residue is allowed, or alternatively wherein said first peptide is derived from the peptide sequence MLAVISCAV or MLAVVSCAV in such a way that a) either residue M at position 1 and residue A at position 8 remain unchanged, or that b) residue L at position 2 and residue V at position 9 remain unchanged,
and that one, two or three, more preferably one or two, even more preferably one, of the other amino acid residues are exchanged for another amino acid residue, preferably wherein the amino acid exchange is a semi-conservative exchange, more preferably wherein the amino acid exchange is a conservative exchange, and/or wherein one or two amino acid residues can be inserted or deleted at any position between amino acid residue 1 and 8 in the case of alternative a) or amino acid residue 2 and 9 in the case of alternative b); wherein preferably said insertion is an insertion of one or two, more preferably one, amino acid residues at position 5 or 6, or wherein preferably said deletion is a deletion of one or two, more preferably one, amino acid residues at position 5 or 6; and/or a composition comprising said peptide sequence to a subject. It is preferred that the subject is a human subject. Preferably said peptide and/or a composition comprising said peptide sequence is administered to a child below age 2 years, preferably to a baby below age 1 year. The method of vaccinating of the invention will provide the vaccinated subject with the desired immune protection. The method of vaccinating of the invention relates to the generation of a para-immune response, that is, to use an example, the yellow fever vaccine is not used for the prevention of yellow fever, but for the prevention of the other diseases mentioned, for example for the prevention of melanoma. Thus, in general, the vaccine against disease X, when used in accordance with this invention, is used for the prevention of the other diseases mentioned herein, for example, and in particular, for the prevention of melanoma, but not for the prevention of disease X.

The invention further relates to a peptide wherein either I) said peptide has at least 5, more preferably 6 and most preferably 7, amino acid residues in common with SEQ ID NO:1 when aligned with SEQ ID NO:1 to maximize the number of identical residues in such a way that only at position 5 and position 6 an insertion or deletion of one amino acid residue is allowed, or alternatively II) wherein said peptide is derived from the peptide sequence MLAVISCAV or MLAVVSCAV in such a way that a) either residue M at position 1 and residue A at position 8 remain unchanged, or that b) residue L at position 2 and residue V at position 9 remain unchanged,
and that one, two or three, more preferably one or two, even more preferably one, of the other amino acid residues are exchanged for another amino acid residue, preferably wherein the amino acid exchange is a semi-conservative exchange, more preferably wherein the amino acid exchange is a conservative exchange, and/or wherein one or two amino acid residues can be inserted or deleted at any position between amino acid residue 1 and 8 in the case of alternative a) or amino acid residue 2 and 9 in the case of alternative b); wherein preferably said insertion is an insertion of one or two, more preferably one, amino acid residues at position 5 or 6, or wherein preferably said deletion is a deletion of one or two, more preferably one, amino acid residues at position 5 or 6; for use in vaccination for the prevention of a malignancy, wherein the peptide does not have the peptide sequence SEQ ID NO.1. Preferably said peptide is selected from the group consisting of SEQ ID NO: 3-97, more preferably selected from any one from the following groups consisting of SEQ ID NO: 3-13 or SEQ ID NO: 14-24 or SEQ ID NO: 25-35 or SEQ ID NO: 36-46 or SEQ ID NO: 47-57 or SEQ ID NO: 58-67 or SEQ ID NO: 68-77 or SEQ ID NO: 78-87 or SEQ ID NO: 88-97.

In a further preferred embodiment, said peptide has a length of from 8 to 12 amino acid residues. Peptide synthesis is know in the art. (W. Chang, P. D. White ; Fmoc solid phase peptide synthesis, a practical approach; Oxford University Press, Oxford, 2000, ISBN 0199637245).

In a further preferred embodiment, the peptide is further coupled to a carrier polypeptide. Examples of carrier polypeptides useful in the present invention are preferably polypeptides derived from tetanus or diphtheria toxoid, or choleratoxin, the outer membrane protein complex CRM197, hepatitis B surface antigen (HBsAg) or hepatitis B core antigen, or for example mutants thereof. Coupling can be, for example by a chemical crosslink, for example which crosslink further comprises a spacer, wherein the spacer is selected from the group consisting of functional cross-linkers, flexible amino acid linkers, like the hinge region of immunoglobulins, glycine serine linkers and glycine linkers and homo-bifunctional crosslinkers. For the present invention, preferred linkers are hetero-bifunctional cross-linkers. Several hetero-bifunctional cross-linkers are known to the art. These include the preferred cross-linkers SMPH (Pierce), Sulfo-MBS, Sulfo-EMCS, Sulfo-GMBS, Sulfo-SIAB, Sulfo-SMPB, Sulfo-SMCC, SVSB, SIA and other cross-linkers available for example from the Pierce Chemical Company (Rockford, IL, USA). A preferred cross-linker has one functional group reactive towards amino groups and one functional group reactive towards cysteine residues. The above mentioned preferred cross-linkers all lead to formation of a thioether linkage. Another class of cross-linkers suitable in the practice of the invention is characterized by the introduction of a disulfide linkage between the peptide of the invention and the carrier protein. Preferred cross-linkers belonging to this class include for example SPDP and Sulfo-LC-SPDP (Pierce).

Examples of flexible spacers useful for crosslinking are are the hinge region of Immunoglobulins, glycine serine linkers (GGGGS)ₙ, and glycine linkers (G)ₙ.

It is further preferred that the peptide is capable of being presented by the HLA A2 or another frequently occurring HLA class I molecule. For example, the peptide may comprise an anchor sequence for presentation by HLA A2 class I.

The invention further relates to a polypeptide comprising a peptide sequence as defined in I) or II) above 5 for use in vaccination for the prevention of a malignancy, wherein said polypeptide is a fusion polypeptide which is not a naturally occurring polypeptide. Polypeptides of the invention are obtainable, for example, by way of recombinant protein expression. Recombinant polypeptides are obtainable by way of standard molecular biological techniques as, for example, described in Molecular Cloning: A Laboratory Manual, 3rd edition, Eds. Sambrook et al., CSHL Press 2001. Briefly, polypeptides can be expressed from recombinant expression vectors comprising a nucleic acid encoding the desired polypeptide, which nucleic acid is operably linked to at least one regulator sequence allowing expression of the desired polypeptide. For example, a nucleic acid sequence encoding the desired polypeptide can be isolated and cloned into an expression vector and the vector can then be transformed into a suitable host cell for expression of the desired polypeptide. Such a vector can be a plasmid, a phagemid or a cosmid. For example, a nucleic acid molecule can be cloned in a suitable fashion into prokaryotic or eukaryotic expression vectors (Molecular Cloning, see above). Such expression vectors comprise at least one promoter and can also comprise a signal for translation initiation and - in the case of prokaryotic expression vectors - a signal for translation termination, while in the case of eukaryotic expression vectors preferably expression signals for transcriptional termination and for polyadenylation are comprised. Examples for prokaryotic expression vectors are, for expression in Escherichia coli e.g. expression vectors based on promoters recognized by T7 RNA polymerase as described in US 4,952,496, for eukaryotic expression vectors for expression in Saccharomyces cerevisiae, e.g. the vectors G426/Met25 or P526/Gall (13), for the expression in insect cells, e.g. Baculovirus vectors, as e.g described in EP-B1-0127839 or EP-B1-0549721 or by Ciccarone et al. ("Generation of recombinant Baculovirus DNA in E.coli using baculovirus shuttle vector" (1997) Volume 13, U. Reischt, ed. (Totowa, NJ: Humana Press Inc.) and for the expression in mammalian cells, e.g. the vectors Rc/CMW and Rc/ASW and sw40-Vectors, which are commonly known and commercially available, or the EBNA-system described in Example 4, the Sindbis replicon-based pCytTS (14), the Sindbis virus expression system (15) or an Adenovirus expression system (16). The molecular biological methods for the production of these expression vectors as well as the methods for transfecting host cells and culturing such transfected host cells as well as the conditions for producing and obtaining the polypeptides of the invention from said transformed host cells are well known to the skilled person.

The invention further relates to a polypeptide for use in vaccination for the prevention of a malignancy wherein said polypeptide comprises a peptide sequence which peptide sequence is selected from the group consisting of SEQ ID NO: 3-97, for example selected from any one from the following groups consisting of SEQ ID NO: 3-13 or SEQ ID NO: 14-24 or SEQ ID NO: 25-35 or SEQ ID NO: 36-46 or SEQ ID NO: 47-57 or SEQ ID NO: 58-67 or SEQ ID NO: 68-77 or SEQ ID NO: 78-87 or SEQ ID NO: 88-97. Preferably the polypeptide is derived from Mycobacterium bovis, Mycobacterium tuberculosis, vaccinia virus, Staphylococcus aureus, yellow fever virus, Chlamydia pneumoniae, Chlamydia trachomatis, Entamoeba histolytica, Giardia lamblia, Haemophilus ducreyi, Japanese encephalitis B, Klebsiella pneumoniae, Leishmania donovani, Moraxella catarrhalis, Mycobacterium leprae, Pasteurella multocida, Pseudomonas aeruginosa, Salmonella typhi, Serratia marcescens, Shigella flexneri, Treponema pallidum, Venezuelan equine encephalitis virus, Vibrio cholerae, Vibrio parahaemolyticus, Yersinia enterocolitica, Bartonella bacilliformis, Bordetella pertussis, Brucella mellitensis, Fusobacterium nucleatum, Haemophilus influenzae, Leishmania major, Listeria monocytogenes, Neisseria gonorrhoe, Neisseria meningitides, orf-virus, Pasteurella multocida, Pneumocystis carinii, Proteus vulgaris, Pseudomonas aeruginosa Rickettsia rickettsii, Salmonella typhi, Salmonella enterica, Streptococcus agalactiae, Streptococcus pyogenes, Tick-bome-encephalitis virus, Trichomonas vaginalis, Toxoplasma gondii, Trypanosoma gambiense, Yersinia pestis, Ajellomyces capsulatus, Babesisa bovis, Bacillus anthracis, Bacteroides thetaiotaomicron, Bacteroides fragilis, campylobacter jejuni, Clostridium tetani, Corynebacterium diphtheriae, Enterococcus faecalis, Enterococcus faecium, Escherichia coli, Giardia lamblia, Helicobacter pylori, Legionella pneumophila, Leptospira interrogans, Listeria innocua, Molluscum contagiosum, Mycobacterium fortuitum-chelonae, Mycobacterium marinum, Mycobacterium leprae, Mycoplasma penetrans, Pasteurella multocida, Plasmodium falciparum, respiratory syncytial virus, Tropheryma whippeli, Trypanosoma brucei, Trypanosoma cruzi, Vibrio parahaemolyticus, Vibrio vulnificus, Yersinis pestis,
more preferably wherein the polypeptide is derived from Mycobacterium bovis, Mycobacterium tuberculosis, vaccinia virus, Staphylococcus aureus, yellow fever virus, Chlamydia pneumoniae, Chlamydia trachomatis, Entamoeba histolytica, Giardia lamblia, Haemophilus ducreyi, Japanese encephalitis B, Klebsiella pneumoniae, Leishmania donovani, Moraxella catarrhalis, Mycobacterium leprae, Pasteurella multocida, Pseudomonas aeruginosa, Salmonella typhi, Serratia marcescens, Shigella flexneri, Treponema pallidum, Venezuelan equine encephalitis virus, Vibrio cholerae, Vibrio parahaemolyticus and Yersinia enterocolitica,
most preferably wherein the polypeptide is derived from Mycobacterium bovis, Mycobacterium tuberculosis, vaccinia virus, Staphylococcus aureus and yellow fever virus. The sequence identification numbers of exemplary polypeptides are given in Table 1, but the polypeptides of the invention can also be identified by a simple protein BLAST search for short, nearly exact matches (http://www.ncbi.nlm.nih.gov/BLASTI) using the peptide sequences of any one of SEQ ID NO:3-97 of the invention as query.

The invention further relates to a composition comprising a peptide or polypeptide of the invention as defined above, further comprising an adjuvant, for use in vaccination for the prevention of a malignancy. There are several technologies for increasing the immunogenicity of peptides. Already mentioned was the genetic fusion of defined epitopes to a carrier protein that forms large particles to improve the presentation of the peptide to the cells of the immune system. Fusion partners of this type are HBsAg and the hepatitis B core antigen. Alternatives are to make genetic or chemical fusions with immunogenic proteins such as tetanus toxoid, or to produce a complex peptide as a multimer of the peptide sequence.

The invention further relates to a vaccine suitable for use in children, in particular adapted for use in infants and/or babies, comprising a peptide or polypeptide of the invention as defined above. It is preferred that the vaccine be in unit dosage form, wherein the unit dosage contains the same amount of plaque forming units as the dosage for adults, however, it should be administered in a 0.5 ml volume (17).

A further preferred vaccine comprises two or more different peptides of the invention as defined above, two or more different polypeptides of the invention as defined above, or at least one peptide of the invention as defined above and at least one polypeptide of the invention as defined above.

The invention further relates to a DNA-based vaccine comprising an expression construct comprising a promoter functional in eukaryotic cells and a polynucleotide encoding at least one peptide of the invention as defined above, for example as defined in I) and/or II above, and/or at least one polypeptide of the invention as defined above, for example a polypeptide comprising a peptide of the invention as defined above, for example as defined in I) and/or II above, wherein said polynucleotide is positioned sense to and under the control of said promoter, for use in vaccination for the prevention of a malignancy. DNA based vaccines, for example those comprising a CpG-rich motif or chimaeric bacterial DNA, are advantageously recognized by dendritic cells (antigen-presenting cells [APCs]) and lead to subsequent activation of T lymphocytes. DNA based vaccines lead to both, humoral and comprehensive cell-mediated responses in primates and particularly in a human subject. Administration to human subjects, including children, is in principle regarded as being safe, although the aaplication of such vaccines to humans is postponed until remaining questions on the safety of this kind of vaccine are settled. Of advantage is the generation of strong and high avidity CD8+ cytotoxic T lymphocyte (CTL) responses in primates, after administration of chimaeric DNA followed by a boost with a chimaeric live viral vector. Such chimaeric live viral vectors can be, for example, an attenuated poxvirus or adenovirus.

Generation of DNA based vaccines is discussed, for example, in two reviews (18, 19).

In a preferred embodiment, the DNA based vaccine is in the form of "naked" DNA vaccines (reviewed in 20). In case of "naked" DNA vaccines, the construction of DNA based vaccines comprises the cloning of the polynucleotide encoding at least one peptide of the invention as defined above, for example as defined in I) and/or II above, and/or at least one polypeptide of the invention as defined above, for example a polypeptide comprising a peptide of the invention as defined above, for example as defined in I) and/or II above into an expression cassette, for example under the control of a viral promoter, like the cytomegalovirus immediate early promoter. Without wishing to be bound to any theory, it is believed that "naked" DNA vaccines function by moving to cell nuclei after administration, possibly persisting there and resulting in long-term expression of the encoded proteins by the host's cells, providing a stable and persistent source of the encoded antigen leading to a permanent stimulation of the immune system and the generation of long-lasting immunity. An advantage of DNA immunization is that both cellular (including CD4⁺ and CD8⁺ T cells) and humoral immune responses can be induced. Peptide epitopes encoded by the naked DNA vaccine, for example the peptide of the invention as defined above, for example as defined in I) and/or II above, are presentable by major histocompatibility complexes (MHC) class I as well as class II complexes after processing into peptides by the proteasome. The MHC class I- "peptide of the invention" complex can be recognized by CD8⁺ T cells and can induce CD8⁺ T cells to acquire antigen-specific cytotoxic functions. These CD8⁺ cytotoxic T lymphocytes (CTL) should then be able to kill cells with a peptide of the invention presented on their surface, for example a melanoma cell with HERV-K-MEL on its surface.

As far as administration of "naked" DNA vaccines is concerned, they include direct intramuscular or intradermal injections of expression vectors, administration with a needle-free injection device, administration by a gene gun using gold particles coated with DNA into the target tissue, *in vivo* electropermeabilization, for example by intramuscular administration as polymer-based formulations of DNA followed by electroporation of the injected muscle and the use of aerosols.

In a preferred embodiment, the DNA based vaccine is in the form of the polynucleotide encoding at least one peptide of the invention as defined above, for example as defined in I) and/or II above, and/or at least one polypeptide of the invention as defined above, for example a polypeptide comprising a peptide of the invention as defined above, for example as defined in I) and/or II above into an expression cassette, for example under the control of a viral promoter, like the cytomegalovirus immediate early promoter, introduced into a dendritic cell, preferably an autologous dendritic cell. For the introduction of said polynucleotide into a dendritic cell, a replication-incompetent adenoviral vector comprising the polynucleotide encoding at least one peptide of the invention as defined above, for example as defined in I) and/or II above, and/or at least one polypeptide of the invention as defined above, for example a polypeptide comprising a peptide of the invention as defined above, for example as defined in I) and/or II above into an expression cassette, for example under the control of a viral promoter, like the cytomegalovirus immediate early promoter, is preferred. The immunogenicity of the vaccine can be improved by a formulation of the DNA in cationic lipids (21) or by transfecting dendritic cells (DC) to make use of the DC for professional antigen presentation (22).

In a preferred embodiment, the DNA based vaccine is in the form of a recombinant viral vaccine. In this case the DNA with the expression cassette for the recombinant protein is integrated into a viral genome, the recombinant viral vector. A single round of a self-limited infection with such a recombinant virus can be sufficient to elicit a broad immunity. Therefore, a modified vaccinia virus Ankara (MVA), or a canary-pox virus are preferred candidates. They do not produce infectious virus in the host (23, 24).

For the generation of recombinant viral vaccines several viruses are particularly useful, for example modified vaccinia virus Ankara (MVA), canary-pox-virus, adenovirus, sindbis-Virus, dengue virus, yellow fever virus, measles virus, sendai virus, polio virus, papilloma virus, rota virus, Venezuelan encephalitis virus, semliki forest virus and hepatitis A virus, preferably modified vaccinia virus Ankara (MVA), canary-pox-virus, adenovirus, or sindbis-Virus.

Therefore, the present invention, in a preferred embodiment, further relates to a DNA-based vaccine of the invention may further comprise a polypeptide of modified vaccinia virus Ankara (MVA), canary-pox-virus, adenovirus, sindbis-Virus, dengue virus, yellow fever virus, measles virus, sendai virus, polio virus, papilloma virus, rota virus, Venezuelan encephalitis virus, semliki forest virus and hepatitis A virus. In a further preferred embodiment, the DNA-based vaccine of the invention is in the form of a viral vector, in particular in the form of a modified vaccinia virus Ankara (MVA), canary-pox-virus, adenovirus, sindbis-Virus, dengue virus, yellow fever virus, measles virus, sendai virus, polio virus, papilloma virus, rota virus, Venezuelan encephalitis virus, semliki forest virus and hepatitis A virus. Preferred are vectors of the pox group, papilloma, Venezuelan encephalitis virus, Semliki forest virus, adeno virus and hepatitis A virus. The generation of a DNA-based vaccine based on recombinant viral vaccines of the above mentioned viral types is explained in (23, 24).

In all embodiments relating to a DNA based vaccine of the invention, it is preferred that in the DNA-based vaccine the polynucleotide encodes 5 to 30 copies, preferably 8 to 25, more preferably 10 to 20 copies of the peptide of the invention, for example as defined in I) and/or II above, and/or 2 to 10 copies, more preferably 4-8 copies, of the polypeptide of the invention as defined above, for example a polypeptide comprising a peptide of the invention as defined above, for example as defined in I) and/or II above. Preferably the multiple copies of said peptide or said polypeptide are encoded in the context of a single fusion polypeptide.

The most effective immune responses with designed DNA, DNA-based or peptide-based vaccines are obtained at present by a combination of two different applications. Thus, a prime-boost vaccination regime of DNA either intramuscullary or epidermally, followed by intradermal recombinant modified vaccinia virus Ankara (MVA) is preferred (25).

The following examples are provided to illustrate preferred embodiments of the present invention and are not to be construed to in any way be limiting to the present invention.

### DESCRIPTION OF THE FIGURES

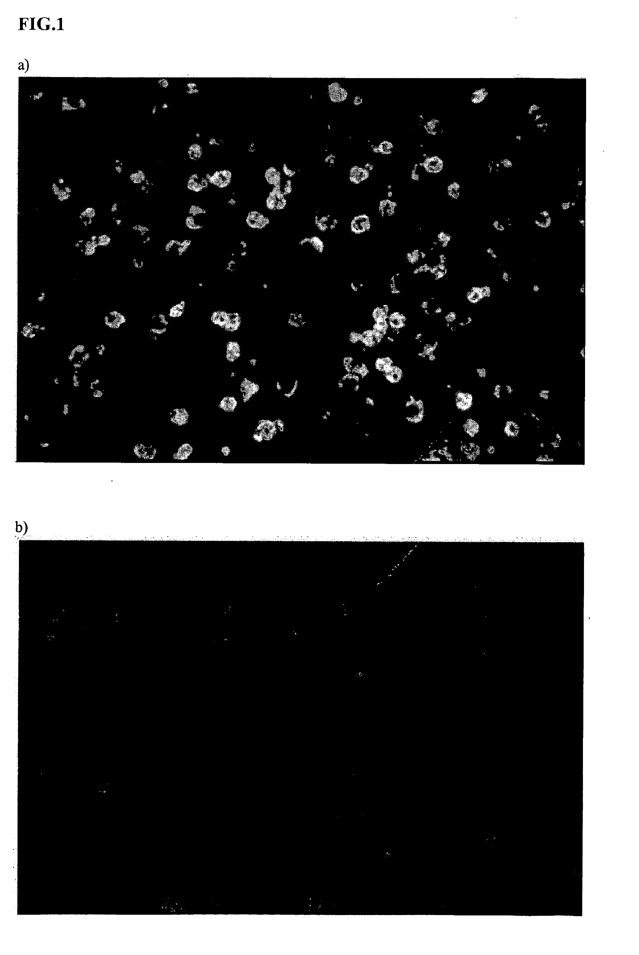

Figure 1 shows the results of an indirect immunofluorescence test using melanoma cells Wörl. In the original experiment pairs of sera from four rhesus monkeys (Macaca mulatta) were investigated; a first serum drawn before vaccination with yellow fever vaccine was compared with a second serum drawn from the same animal four weeks after vaccination with yellow fever vaccine. The sera were tested at a dilution of 1:20, FITC-labelled anti-monkey IgG was used as the secondary antibody. To identify cells in indirect immunofluorescence experiments, melanoma cells were counter-stained with Evans blue. In the original experiments, green FITC fluorescence was only observed in case of the sera obtained from the monkeys four weeks after vaccination with yellow fever vaccine, but not in case of the pre-immune sera of the same monkeys.

Figure 1a shows the result of an indirect immunofluorescence test with melanoma cells Wörl using a serum drawn four weeks after vaccination. Green-fluorescent bound IgG is shown in white.

Figure 1b: shows the result of an indirect immunofluorescence test with melanoma cells Wörl using a serum drawn before vaccination. Red fluorescence of the cell counter stain is shown in grey. No green signal from cell-bound IgG was detected.

### DESCRIPTION OF THE SEQUENCES

SEQ ID NO:1 shows the peptide sequence of HERV K-MEL in the nonapeptide form.
SEQ ID NO:2 shows the peptide sequence of HERV K-MEL in the decapeptide form
SEQ ID NO:3 and following show the peptide sequences in diverse and specified micro-organisms which are pathogenic for humans or are vaccinations for humans.

### EXAMPLES

### Example I

The present inventors found that the two classical vaccinations vaccinia and BCG as well as some mostly severe infectious diseases correlated with a reduced melanoma risk. Using protein blasts for short, nearly exact sequences (NCBI) with the antigen HERV K-MEL, which is present on most melanomas and which is also present already on the presumed melanoma precursors, the congenital and dyslastic naevi cells, the present inventors surprisingly identified polypeptides from 75 micro-organisms which cause diseases in humans. The polypeptides of these micro-organisms have sequence homology to said antigen HERV K-MEL. The present inventors have noted that surprisingly melanoma protective diseases were caused by these micro-organisms. Moreover, surprisingly the identified polypeptides also showed appropriate anchor sequences which are suitable for the presentation together with the HLA A2 class I molecule. The latter is in turn used by the melanoma cells to present the target antigen to the immune system. When the amino acids between the anchor sequences of the target antigen were permutated (10 trials), the present inventors found only 1 or no polypeptide from a human pathogenic micro-organism in an otherwise identical BLAST search with a sequence homology to the permutated sequences (Table 4).

The present inventors noted some vaccines which are currently licensed and which the present inventors consider as outstanding candidates for melanoma prophylaxis because they have the sequence homology for said target antigen HERV-K-MEL. Yellow fever (YF) vaccination is the main candidate. YF has all the properties which can be deduced from the properties of the causative agents of the melanoma protective natural diseases, which have a statistically significant melanoma preventive effect: often causing severe disease with high fever and frequent lethal course.

The present inventors could show that an immune reactivity against melanoma cells was induced by YF vaccination of monkeys. The procedure of vaccination and the drawings of the sera were described in detail previously (26). We used the original sera from the experiments of Niedrig et al. (26). The sera had been stored frozen below -20°C. All animals had also developed antibodies against YF. It was performed with two different melanoma lines. Sera from before vaccination and sera which were drawn from the animals four weeks after vaccination were tested in different dilutions in phosphate-buffered saline, pH = 7.2 (PBS), in indirect immunofluorescence. Dilutions of the sera (1: 10, 1 : 20, 1 : 40, 1: 80, 1: 160, 1: 320 and 1: 640) in PBS were incubated for 30 min with the cells in a moist chamber, thereafter washed three times with PBS and incubated 30 min at 37°C with FITC-labeled Anti-monkey-IgG. The melanoma cells from two different patients (KII and Wörl) were grown in cell cultures and brought to slides. They were used untreated or treated with 10 µmolar 5-azacytidine for 1 or for 2 days. In the experiments with cell line 2 (Wörl) four from four rhesus monkeys seroconverted, with cell line 1 (KII) three of four apes seroconverted. With cell line 1 there was a very weak reaction already with the pre-vaccination sera (though judged as negative in three of four apes) but after vaccination there were clearly positive signals in these three animals (titers 1:≥40). Cell line 1 and the experiment with the missing reaction after vaccination in the fourth ape: In this case the pre-vaccination serum was already low positive (1 : 10 dilution) and there was no boost after vaccination. 5-Azacytidine treatment had no influence on the strength of the fluorescence signals. In the sera of the apes no antibodies of the IgM class against the melanoma cells were detected. The melanoma cells were from two different patients. Both patients had the HLA-A2 antigen.

### DISCUSSION

Specific vaccines against several severe and frequent infectious diseases which lay a heavy burden of disease on various countries are missing today. To be mentioned are, for example, AIDS, malaria, tuberculosis, leprosy, leishmaniosis, other sexually transmitted diseases, such as syphilis, gonorrhea, chlamydia and chankroid (for more examples see footnote of Table 2). It seems possible to reduce the risk of these and other disease by the vaccination(s) of the invention. Moreover, also several micro-organisms causing allergies have the sequence homology with HERV-K-MEL. Thus, it seems possible to use the vaccination of the invention to give a substitute for the protective impact of various cumulative, naturally acquired infectious diseases which have become rare because of improved standards of hygiene. The use of the vaccination(s) of the invention in early childhood is therefore also intended to reduce the frequency of allergic disorders.

It is surprising that *Serratia marcescens,* the main antigen ingredient of Coley's historical vaccine for tumor therapy, has one of the best sequence homologies to the target antigen HERV K-MEL. Only few micro-organisms have better homologies.

It is also surprising that so many human pathogens have this sequence homology. It might be that it has not only a benefit for the human host. The host can use immunologic significant contacts to micro-organisms with said homology to establish a prophylactic non-specific immune response which is permanently available. One can address it as a "natural vaccination". (This, for example, finds a parallel in the strong rejection of other than the own human blood group determinants of the ABO system as experienced by blood transfusions which cause the major transfusion event. This is mediated by antibodies which are induced by micro-organisms in the gut and is based on pre-formed immunologic cross-reactions.)

It should be appreciated that nearly all scientists who are interested in vaccination against cancer look for therapeutic vaccinations. This task is completely different from prophylactic vaccination, because it must focus on antigens of the individual tumor and must induce strong cytotoxic effects. Prophylaxis does not have the same requirements.

The invention provides a vaccination with yellow fever for the whole (at least Caucasian) population in order to reduce melanoma risk and the risk of some other malignancies, as exemplified above. It seems possible to give this vaccination in a way (intranasal), which needs no trained medical personal (26). Thus, during mass vaccination campaigns comparable to oral polio vaccination, one might reduce the costs for manufacturing and application of the vaccine to 2-3 Euro per person as compared with about 35 Euro for an individual vaccination given today to travelers.

Alternatively, the invention provides a vaccination with another of the licensed vaccinations (Table 5), which have the potential to reduce melanoma risk and the risk of some other diseases and malignancies. The invention further provides a vaccination with a new DNA-based or peptide-based vaccination which uses mixtures of DNA-constructs coding for peptides, or the peptides themselves, with the homologous sequences to the HERV K-MEL target antigen (Table 1). The DNA-constructs might be cloned also in a viral vector, such as vaccinia, another pox virus, adenovirus, Sindbis virus or retrovirus. Alternatively, the peptides with said sequence homologies could be coupled to some immunogenic molecule. Moreover, the invention provides a new preventive or therapeutic strategy against one of the pathogenic micro-organisms with said sequence homology, for example a curative therapy of the chronic, widely distributed and severe HIV-infection, the cause of AIDS. A frequent co-infection of HIV is with *Mycoplasma penetrans.* Montagnier, who discovered the HIV, for a long time believed that the *Mycoplasma penetrans* was the true causative agent of AIDS (27). This was, however, not confirmed and the role of the mycoplasmas is not known. Probably they create places of shelter for HIV, where HIV is not attacked by the immune system, because they have the HERV K-MEL homology. A curative therapy of HIV/AIDS is only possible when all active *and latent* viruses become eliminated from the body. *One* necessary measure to achieve this task will be a *bactericidal therapy* of *Mycoplasma penetrans,* for example with gyrase inhibitors, such as the antibiotic ciprofloxacin. Other measures to clear the body from all active or latent HIV must, however, be applied in combination. At present only the highly active anti-retroviral therapy (HAART) against the active HI-viruses is available. Other measures to destroy latent HI-viruses outside of cells and on the surface of cells, such as dendritic cells, are not ready for application to humans.

It is predicted that several thousand cases of melanoma disease (about 100,000 per year world wide) and several thousand cases of death from melanoma (about 20,000 per year world wide) could be avoided annually by the vaccination of the invention. Epidemiological studies indicate that within some decades the melanoma frequency in the developed countries of Europe and North-America will increase to three-times the current numbers because of discontinuing the old vaccinations. Australia, which did not make wide use of the two classical vaccinia and BCG vaccinations as did other countries, already has this high melanoma frequency. Thus, in Europe and North-America the vaccination(s) of the invention may prevent additional 200% melanoma cases and, moreover, may reduce even the frequency of melanoma to about 50% of the cases of today by means of booster vaccinations of older persons. In a rough estimation, each case of disease equals 10,000 Euro, each life which would not be saved by conventional therapy would equal 100,000 Euro. Thus the benefit would be 2.5 x [(10,000 x 100,000) + (100,000 x 20,000)] Euro = 7.5 billion Euro per year. World wide the health systems and the societies could have a profit from the vaccination of the invention of at least about 7.5 billion Euro per year.

The profit should come, for example, from the vaccination of 100 million persons each year. With this strategy all white persons would be vaccinated within a time frame of about 10 years. The vaccination program should continue for the newborns and for a booster of the others every ten years. This means 100 million vaccinations should be given every year in the future. With about 4 Euro per nasal yellow fever vaccination one would have a relation of cost to benefit of 400 million to 7.5 billion Euro, i.e. about 1 to 20. With the proposed strategy this cost-benefit ratio will be reached after ten years. Pre-selection of vaccinees is possible on the basis of the skin type (Fitzpatrick classification type I), the absence of previous vaccinia and BCG vaccination, or on both risk indicators. This procedure would result in an improved cost-benefit ratio at the beginning of the campaign with factors of about 2x, 2x and 6x, respectively. It should be noted that the cost-benefit ratio for the vaccination of the whole population would be comparable to established prophylactic vaccinations against childhood diseases.

The profit will increase since the vaccination(s) (for example with yellow fever vaccine) will protect also against some forms of other malignancies presenting said antigen. Such malignancies include some carcinomas, sarcomas and other malignancies including leukemia as mentioned above. This benefit might even exceed the one of melanoma prophylaxis because of the higher frequency of these other combined malignancies. The profit will increase further since the vaccination(s) provide a para-specific protection against (at least some) other infectious diseases and allergies where the causative agent has also the HERV K-MEL homology. Thus, the course of the infection with (at least some of) these often dangerous infections will be modified. It should be noticed that expositions to these infections result some times in severe and lethal diseases and allergies but lead quite often only to abortive or sub-clinical infections. Probably the latter is favored by a good para-specific immune response. In the developed countries the para-specific immune response is today rarely induced by previous exposure to other micro-organisms. The vaccines of the invention will probably reduce the numbers of severe infectious diseases and of allergies of different origin. The burden of allergies is increasing in the developed world. However, social conditions and standards of hygiene are changing in developing countries. Therefore, in the future these countries will have a great need to induce a para-specific immunity against infectious diseases by means of vaccination(s). In this respect the benefit of the vaccinations might once more exceed even the benefit of the tumor protective effect of the vaccination(s).

Moreover, the knowledge about the identified sequence homologies as outlined opens up the possibility of a curative therapy of HIV/AIDS. *One component* of such a therapy will be a bactericidal therapy of the *Mycoplasma penetrans* co-infection. A curative therapy of HIV would also have an immense economical impact. Other infectious diseases for which the invention might prove useful for prophylaxis or therapeutic intervention include very important infectious diseases of humans such as malaria, tuberculosis, syphilis and many others.

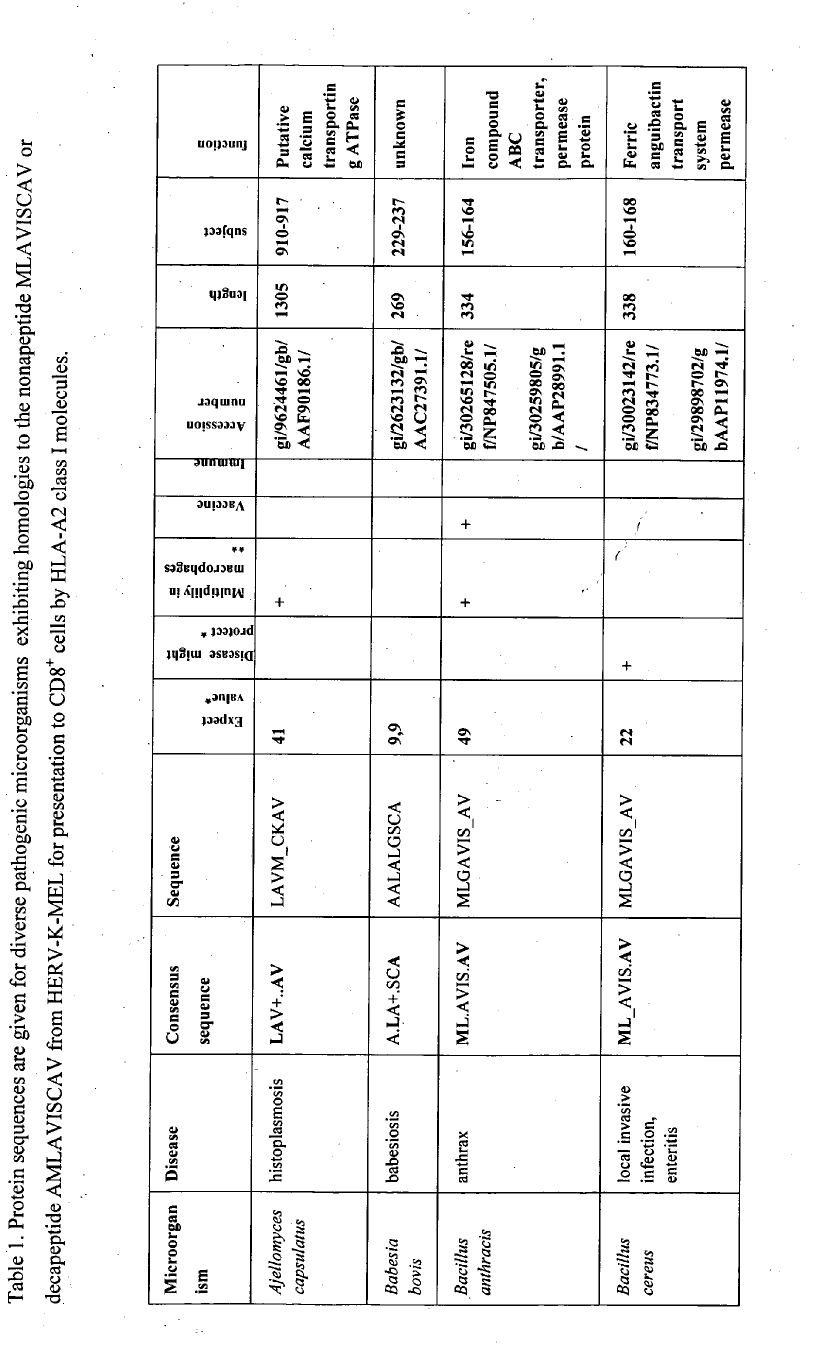

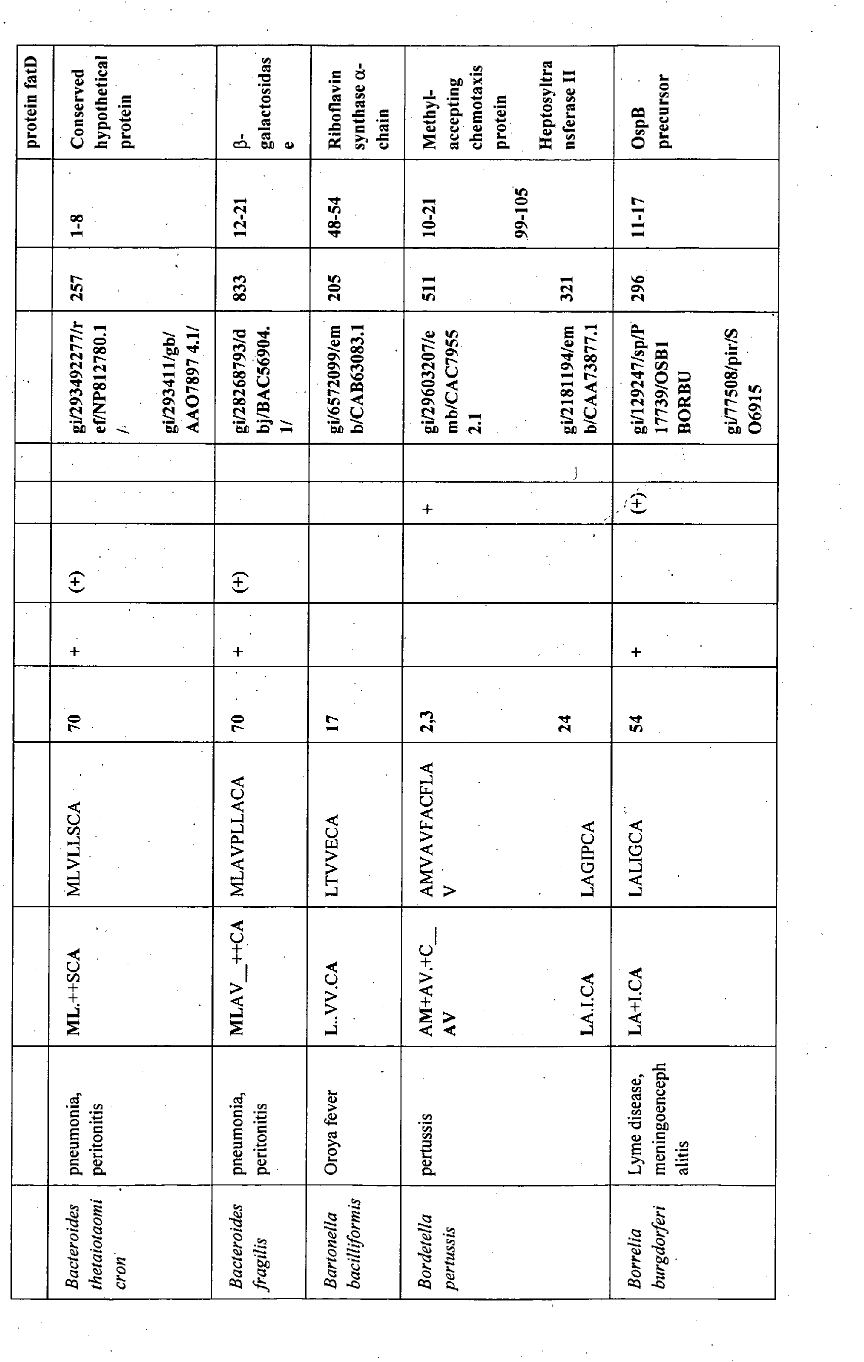

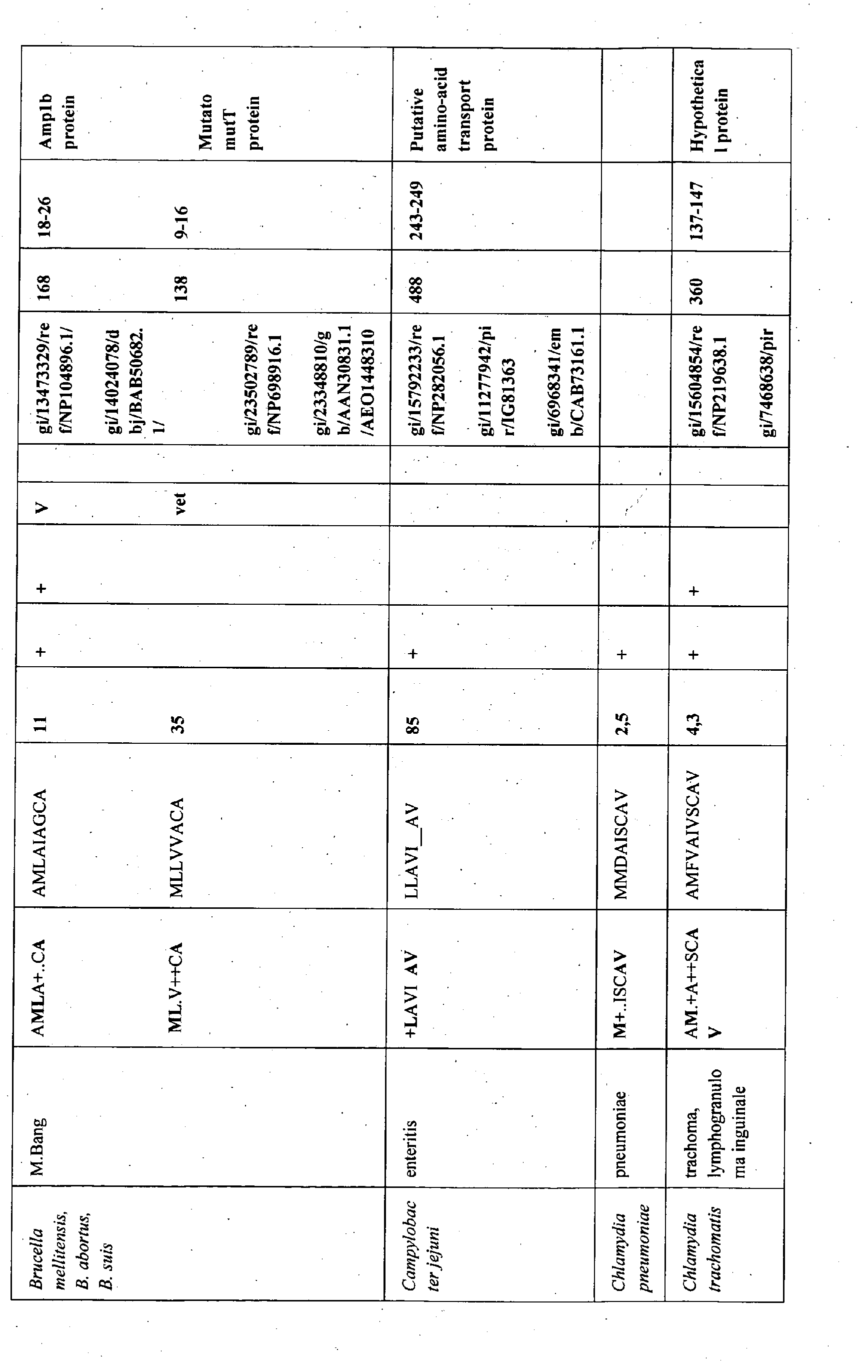

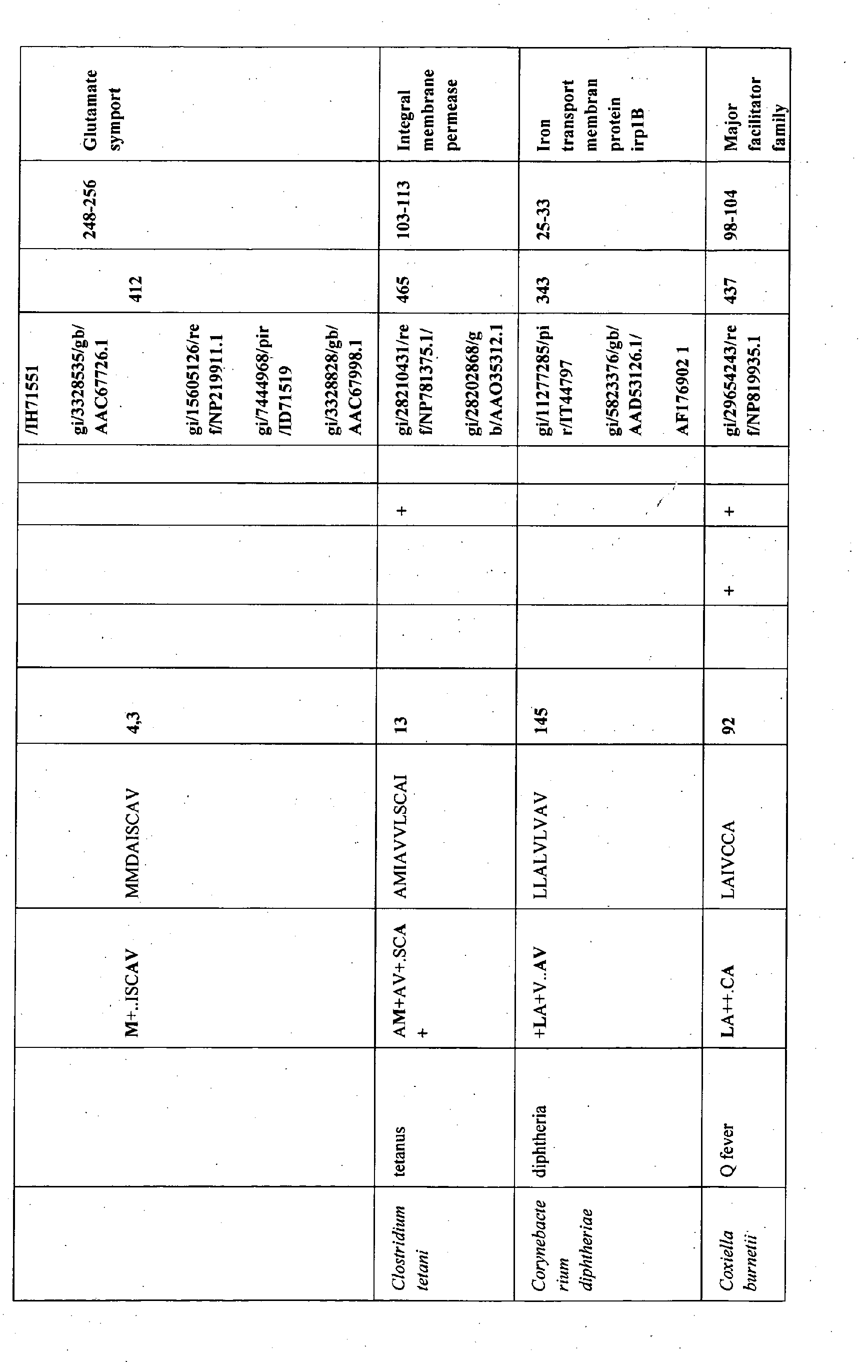

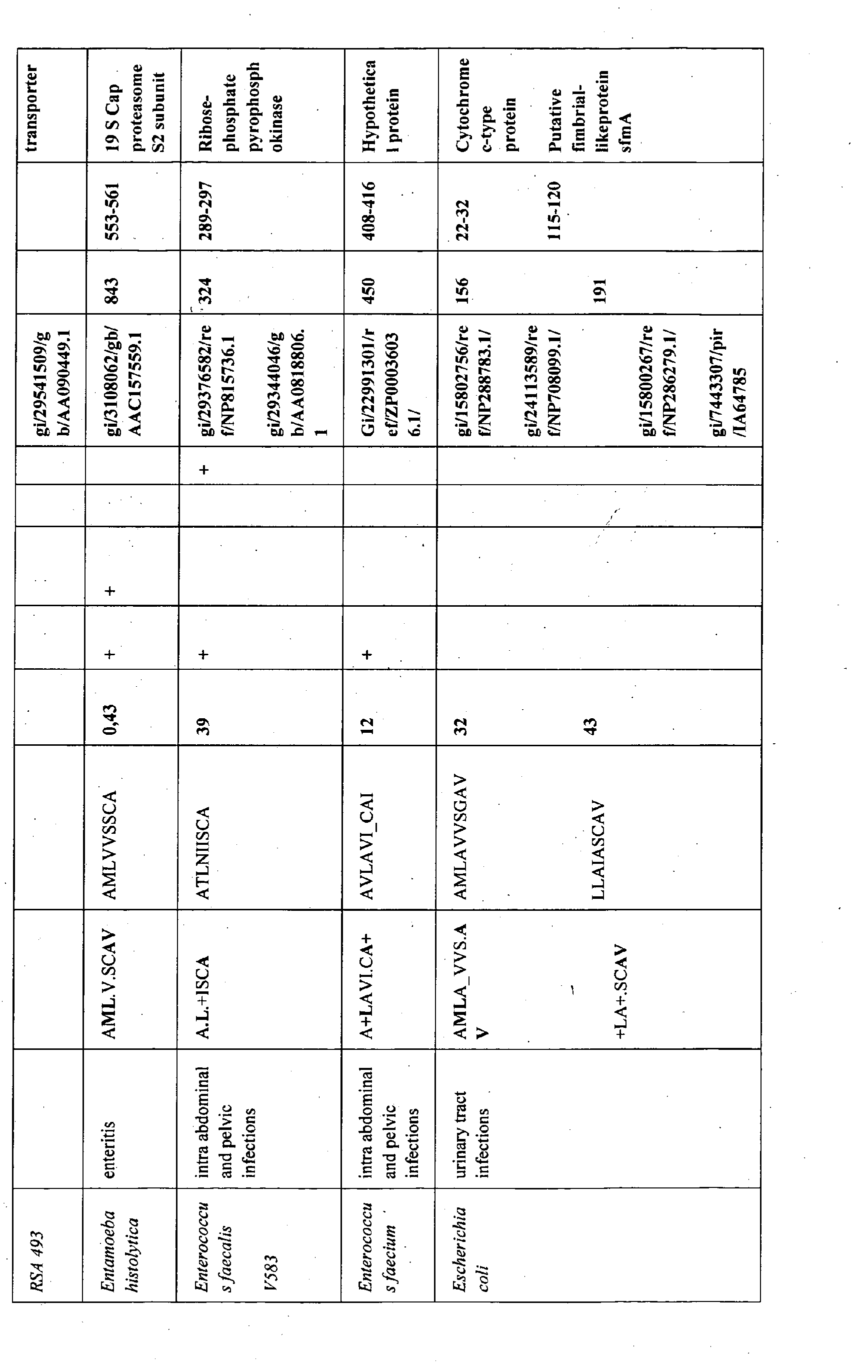

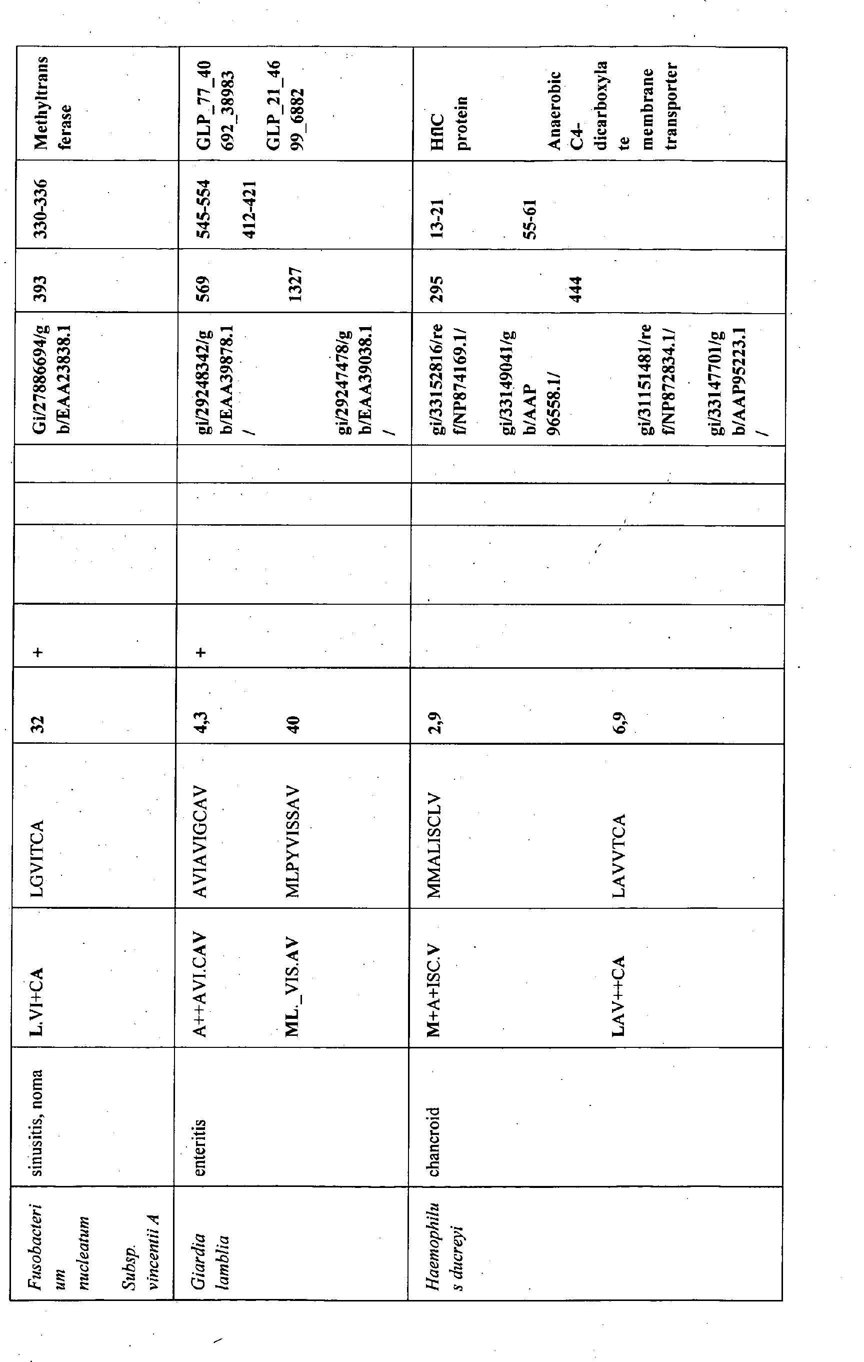

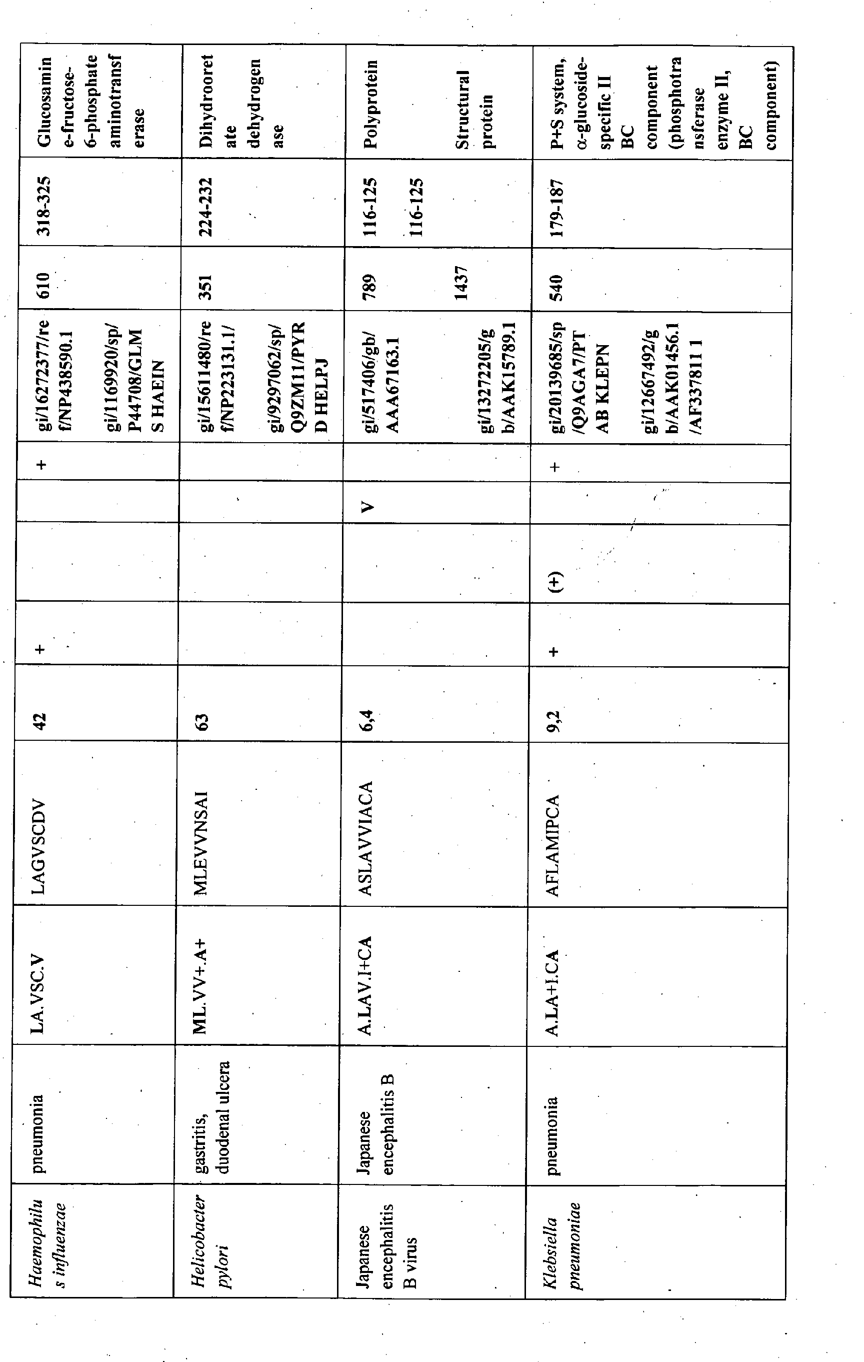

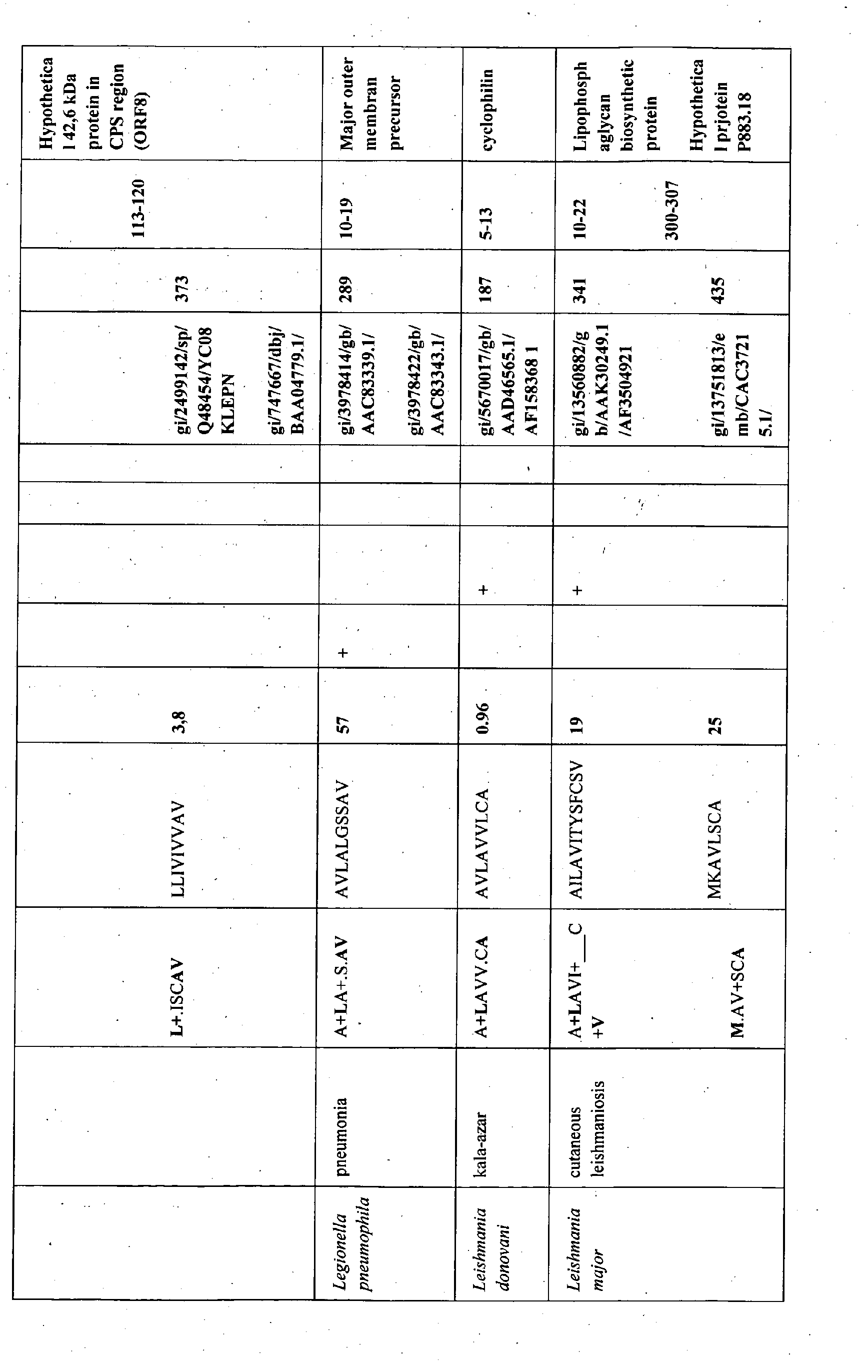

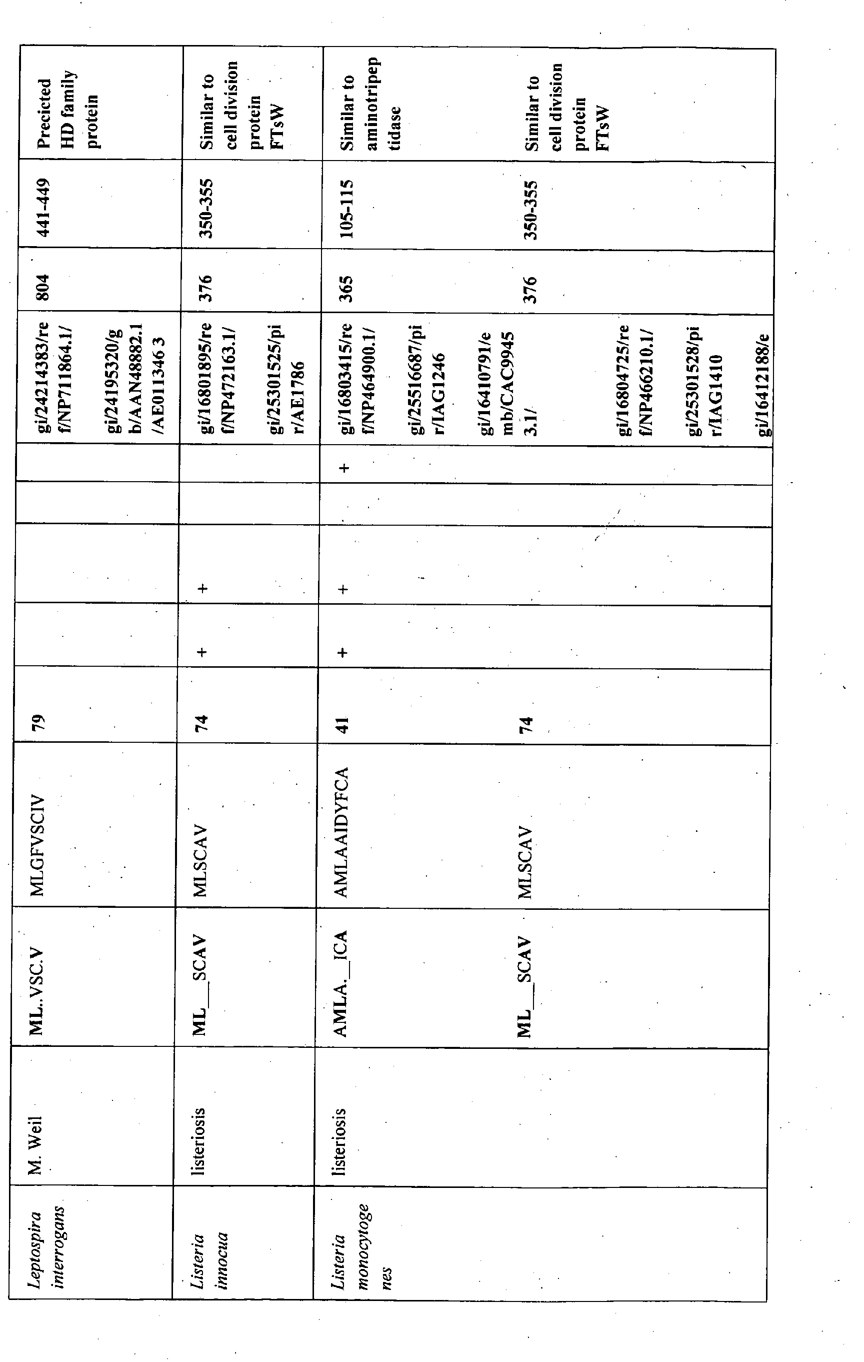

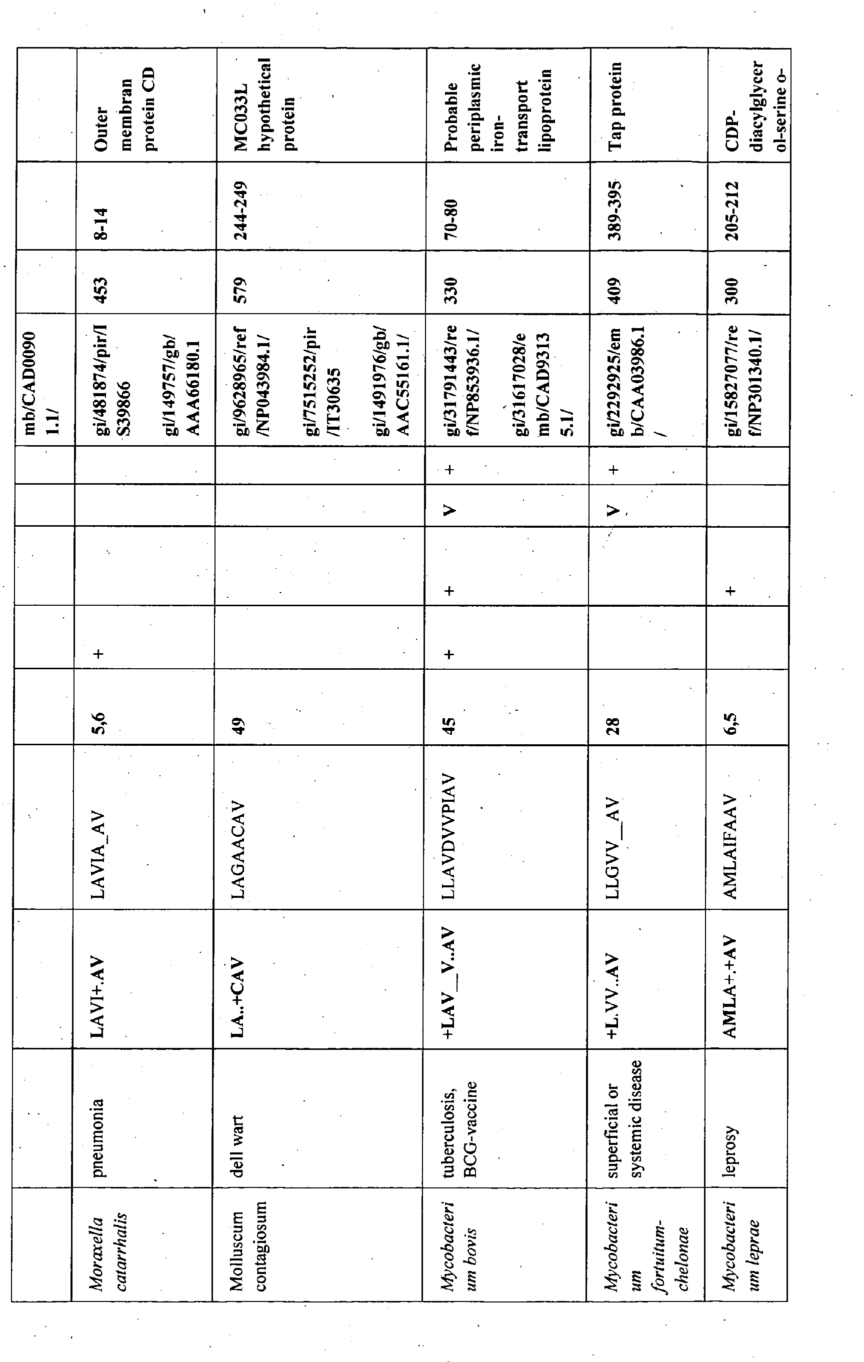

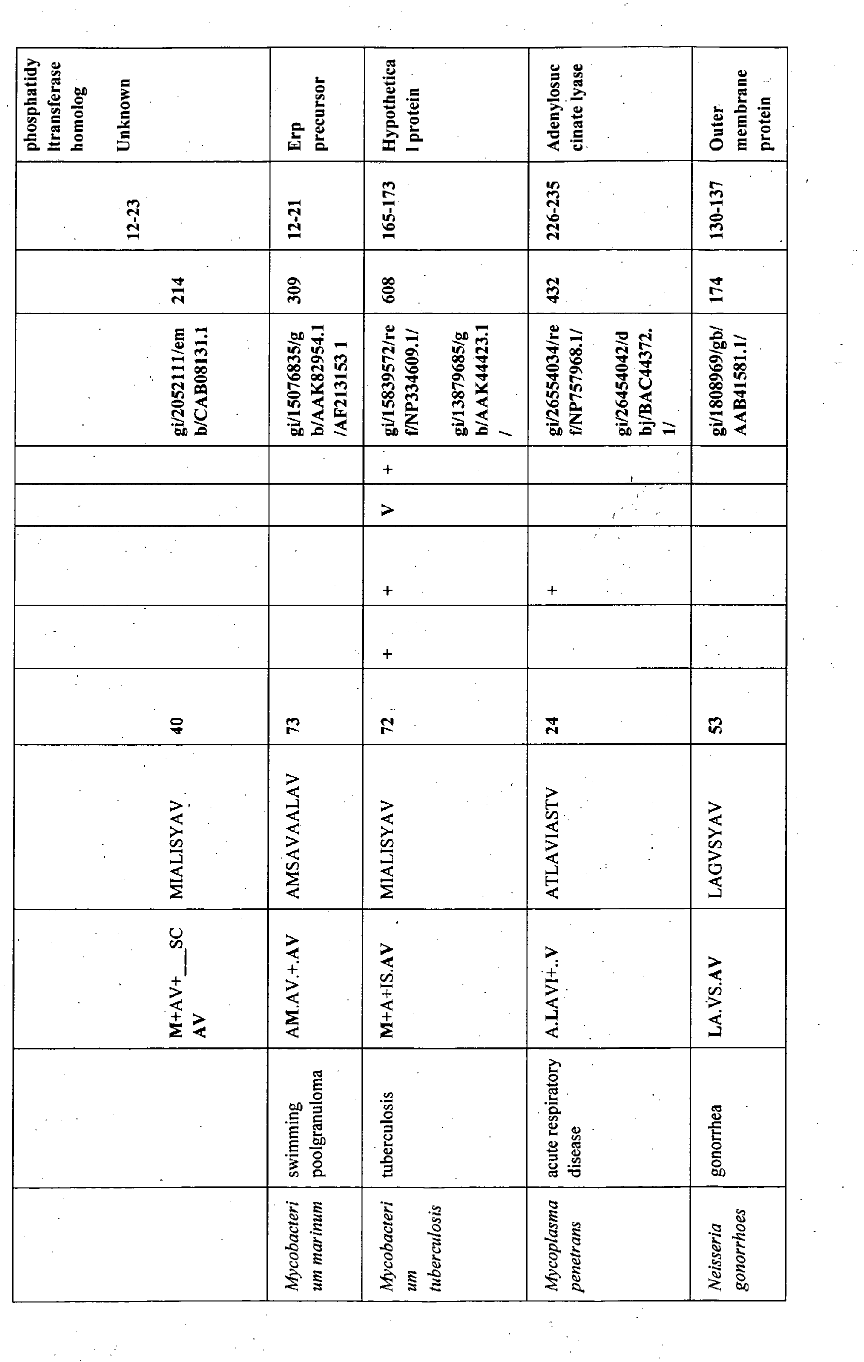

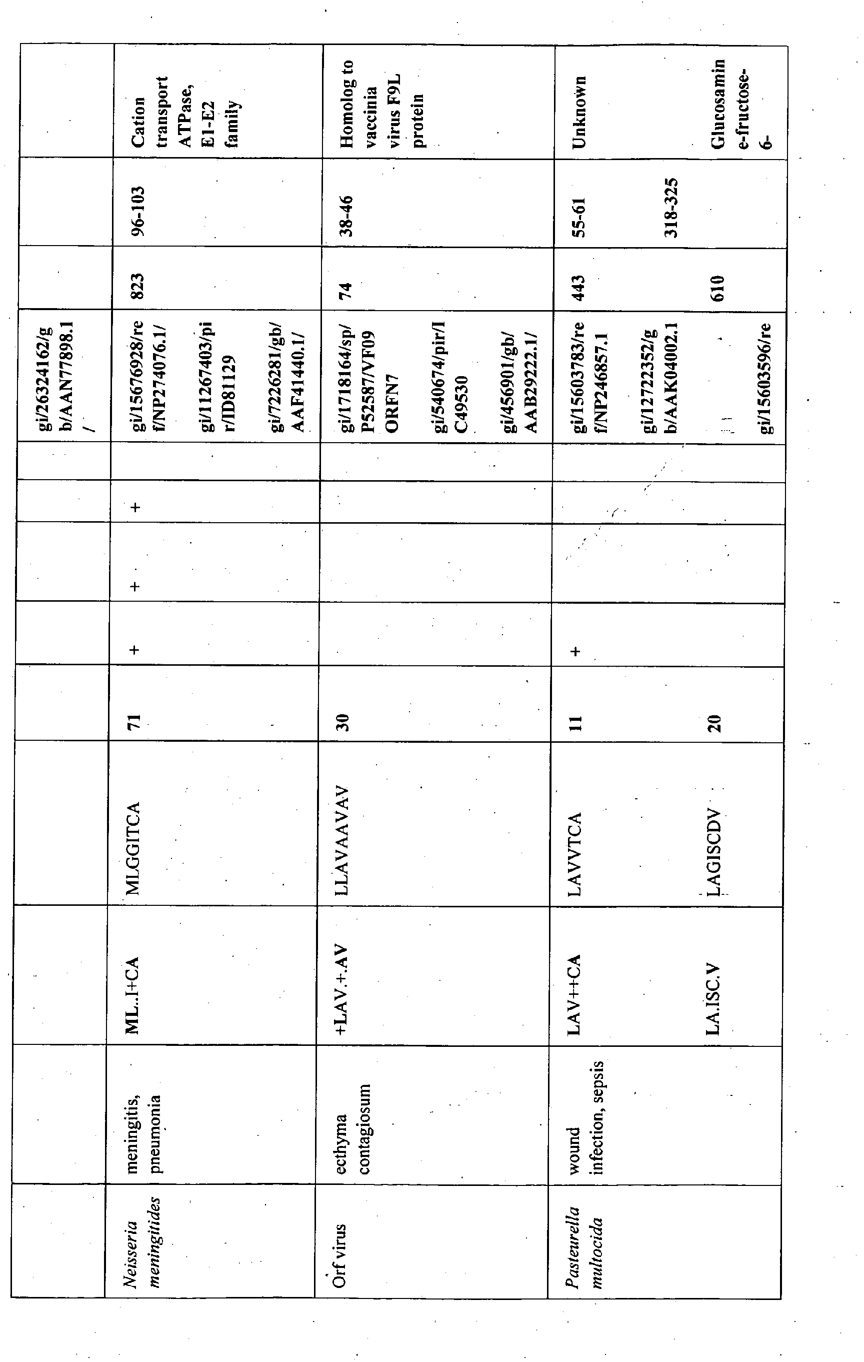

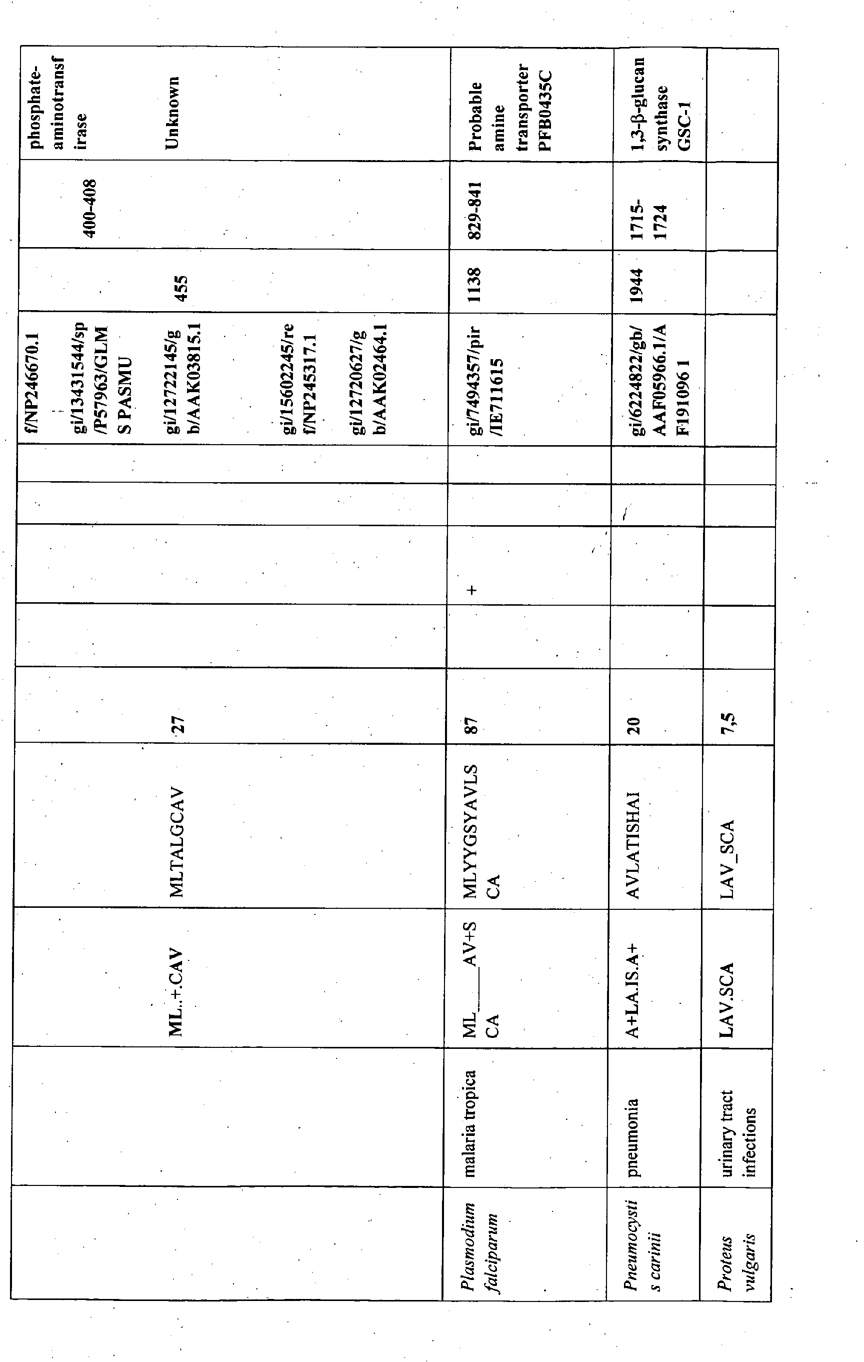

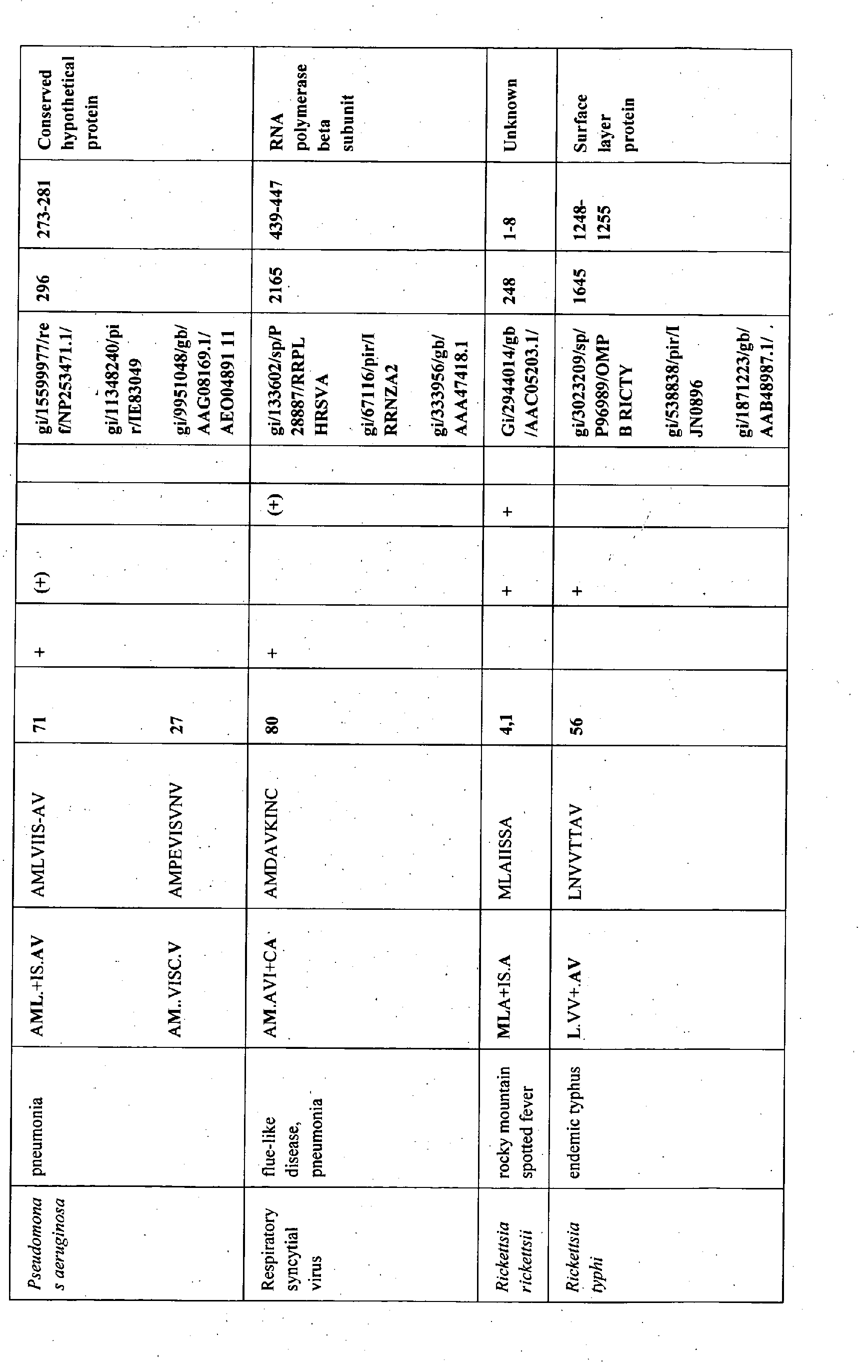

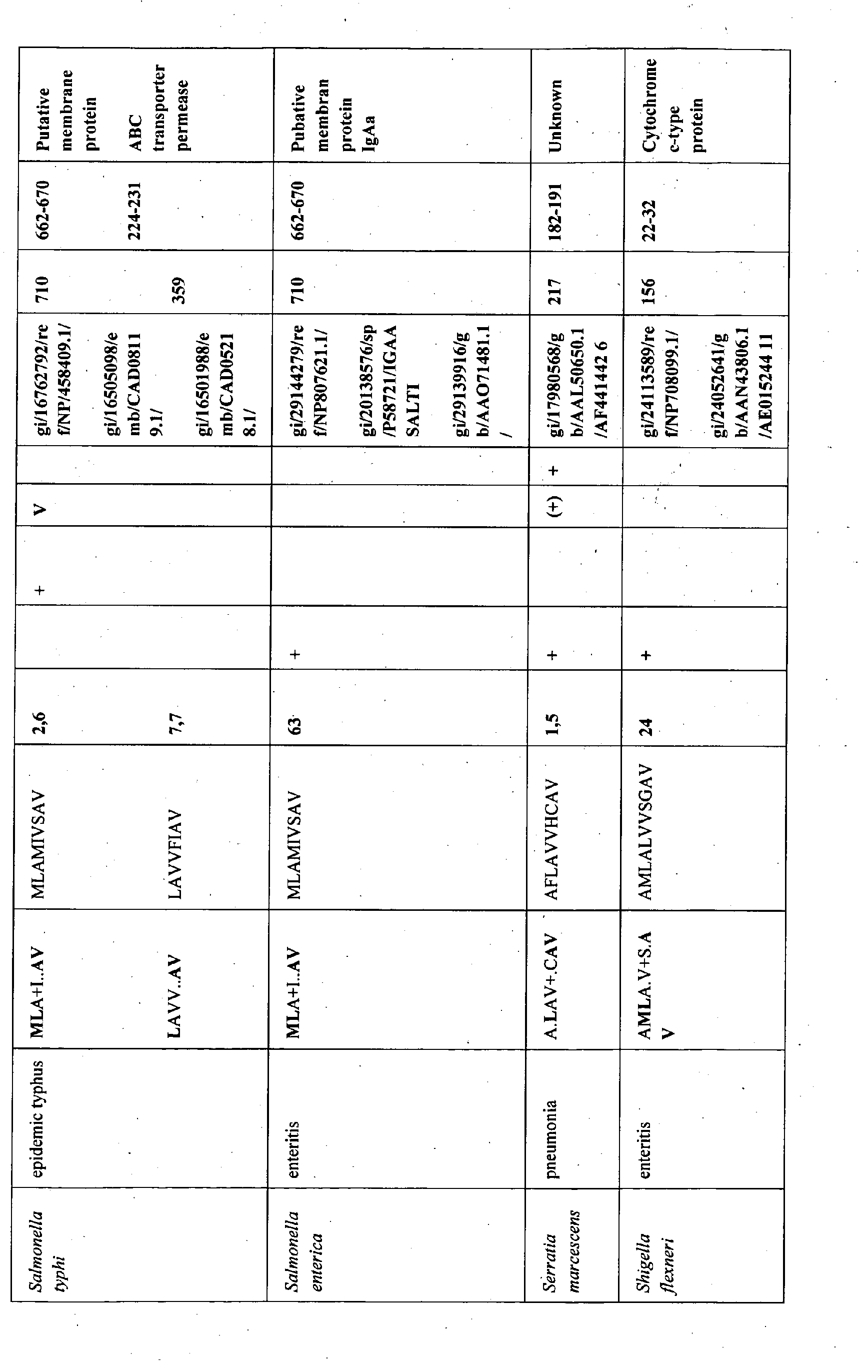

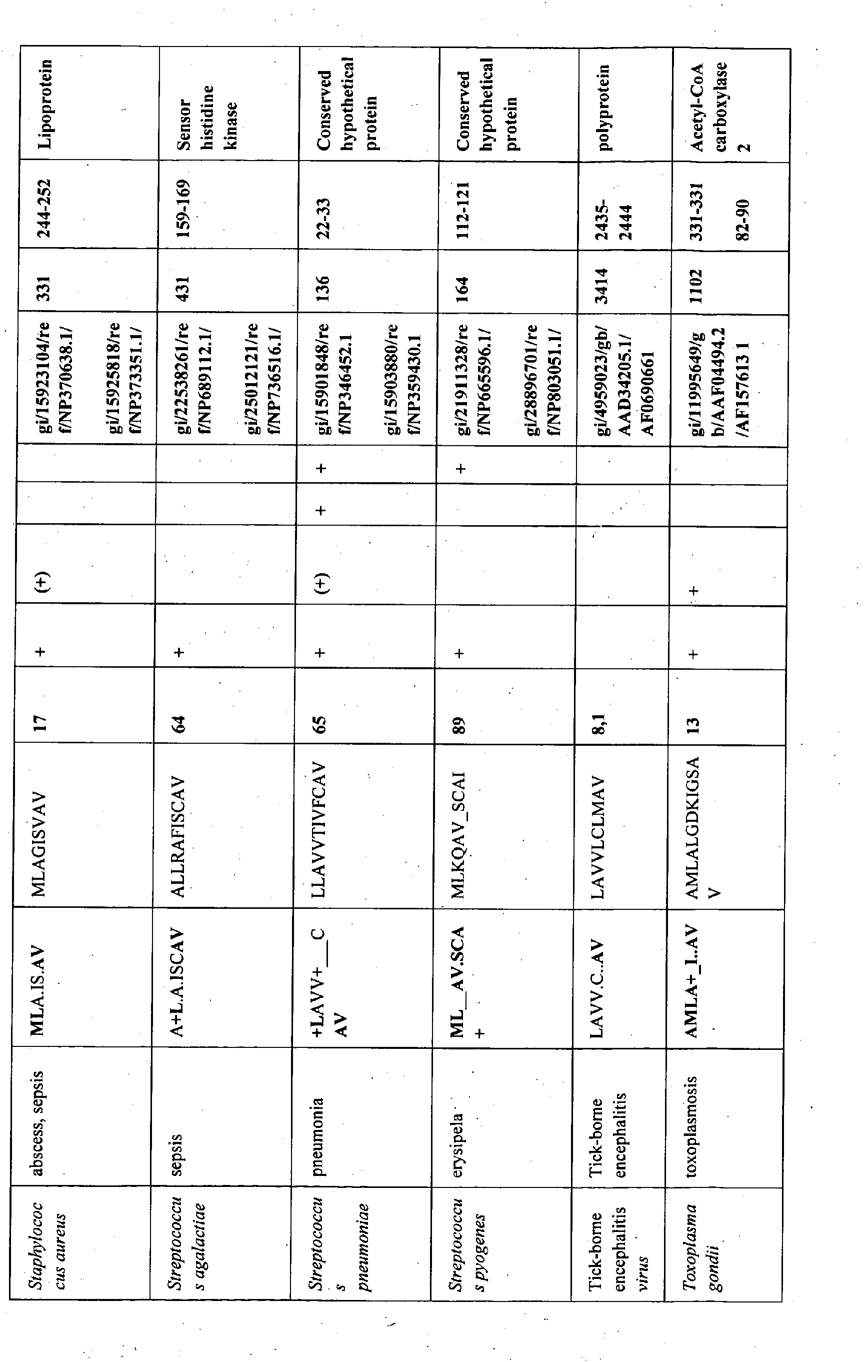

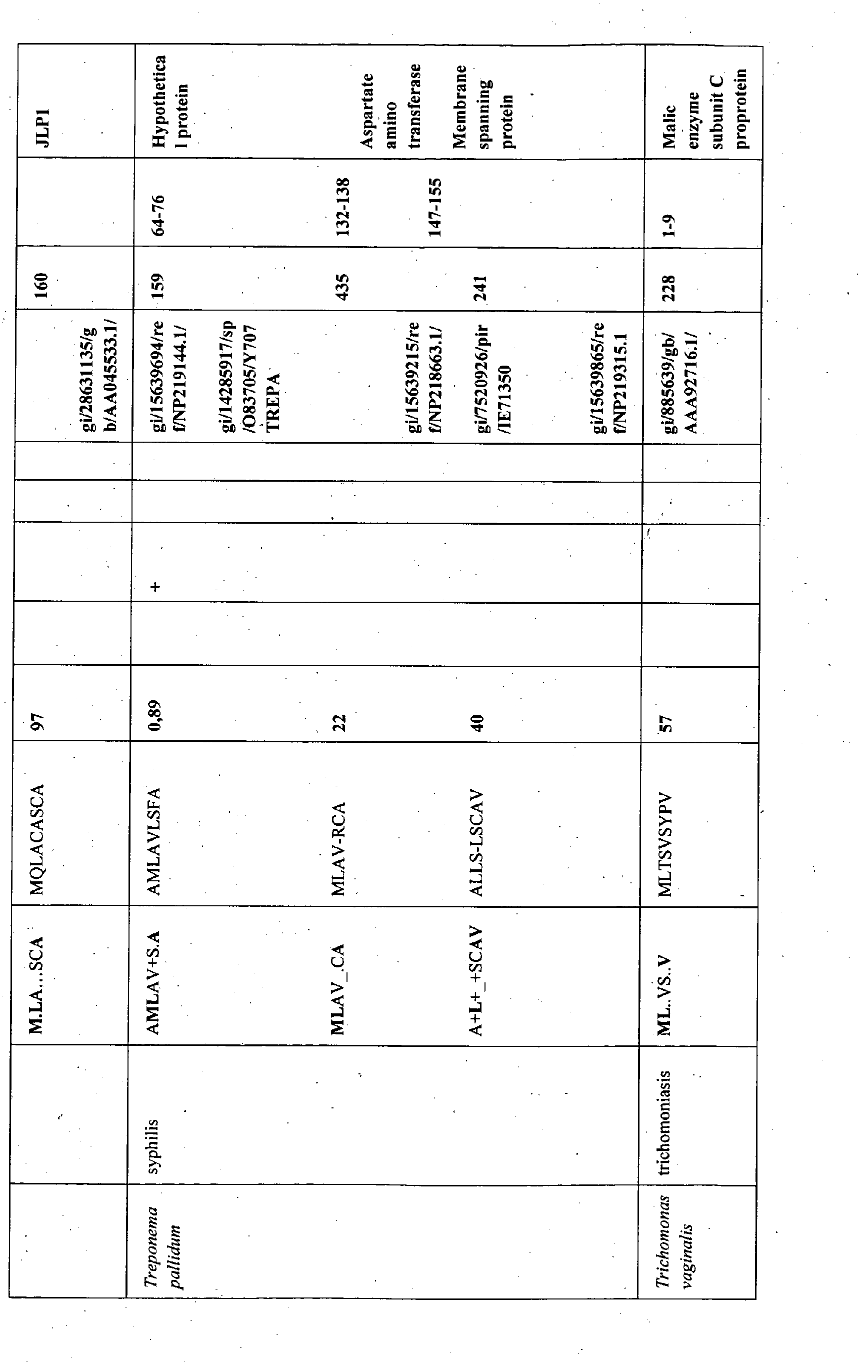

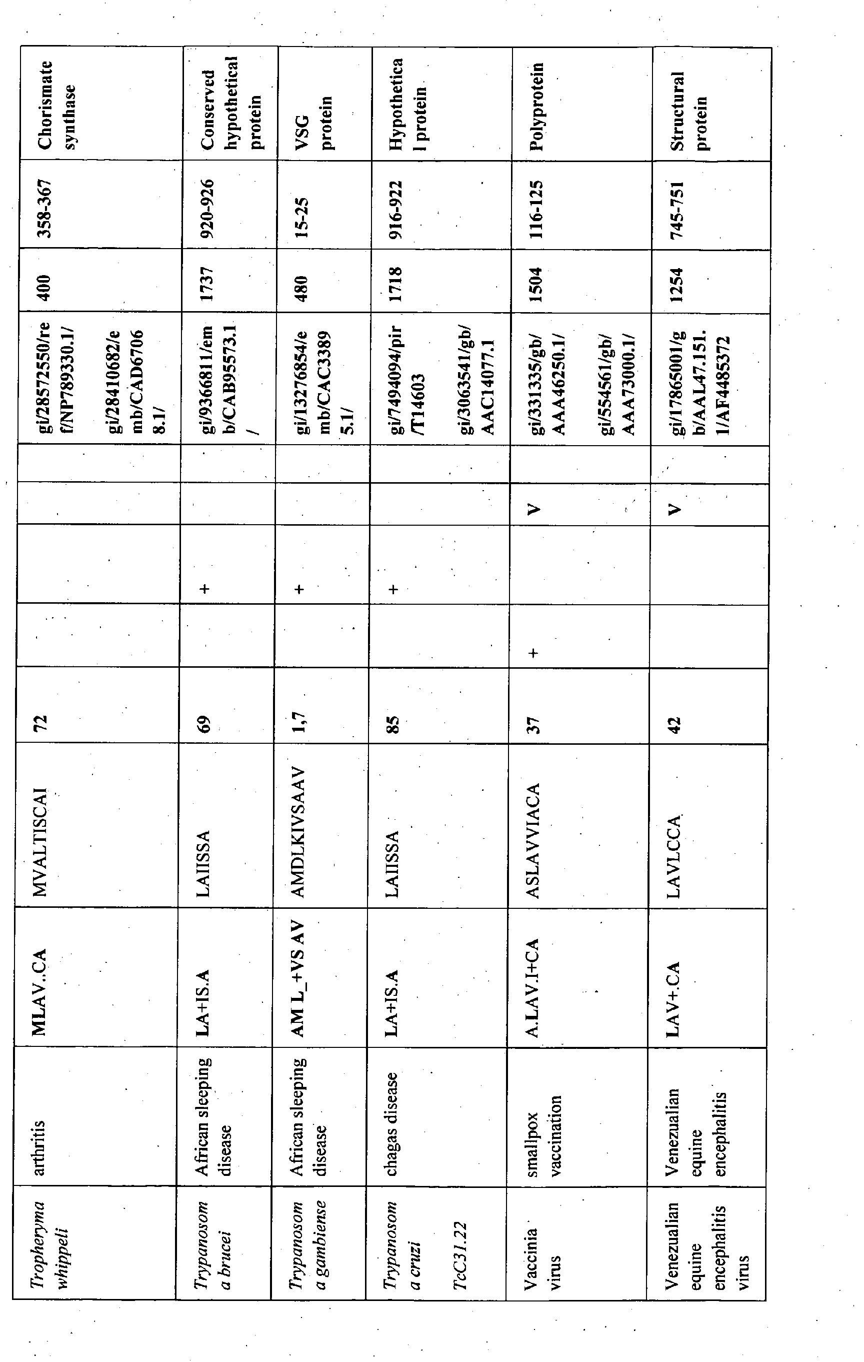

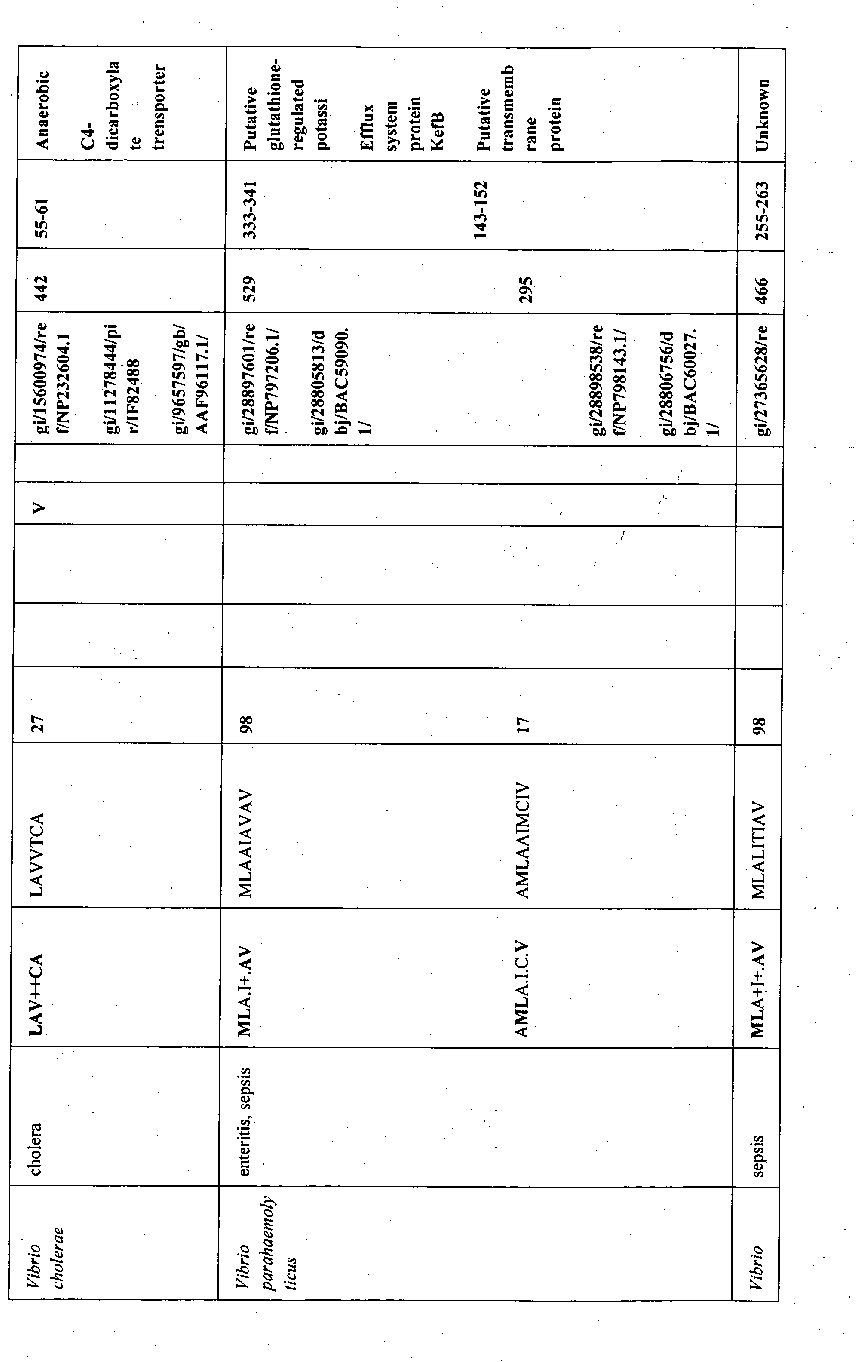

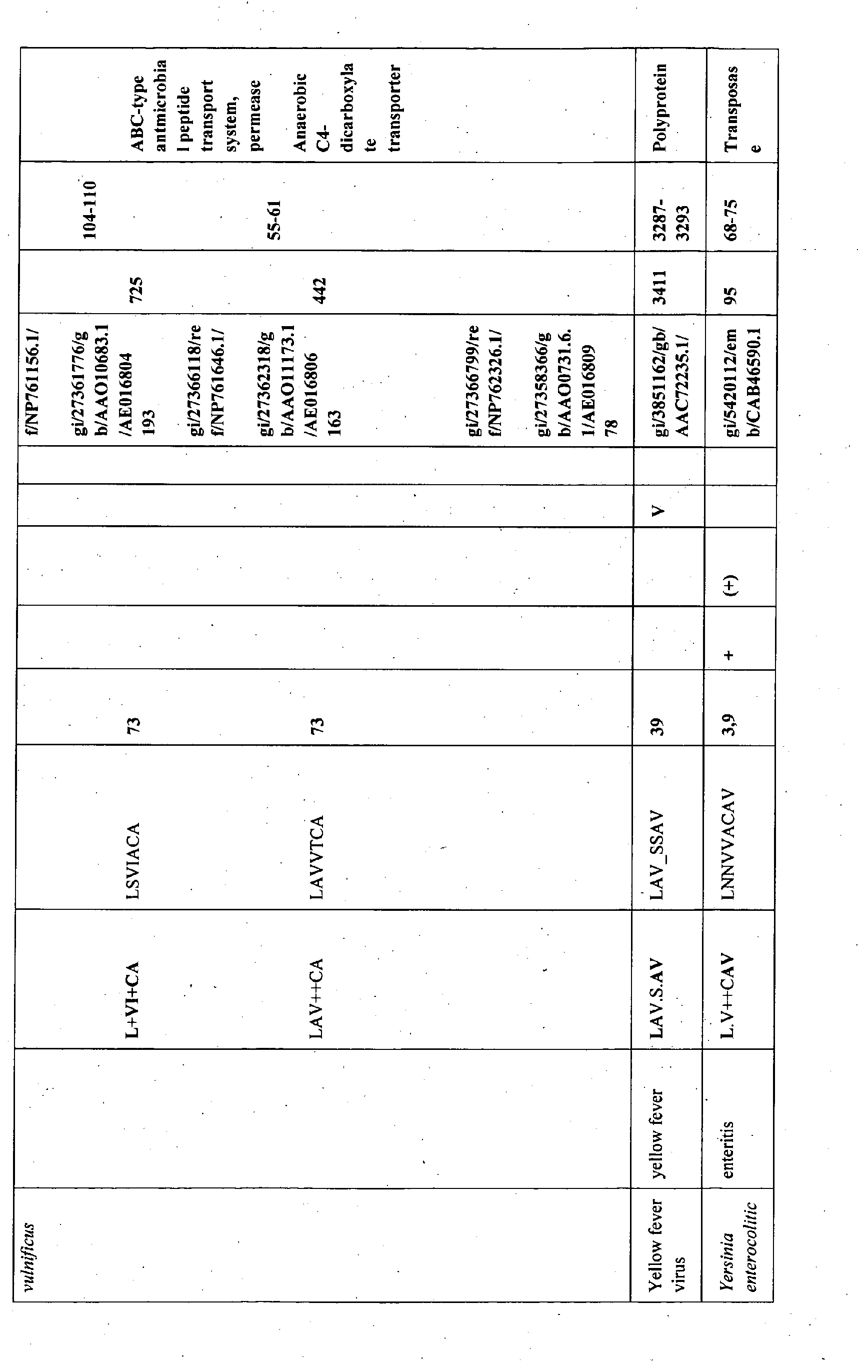

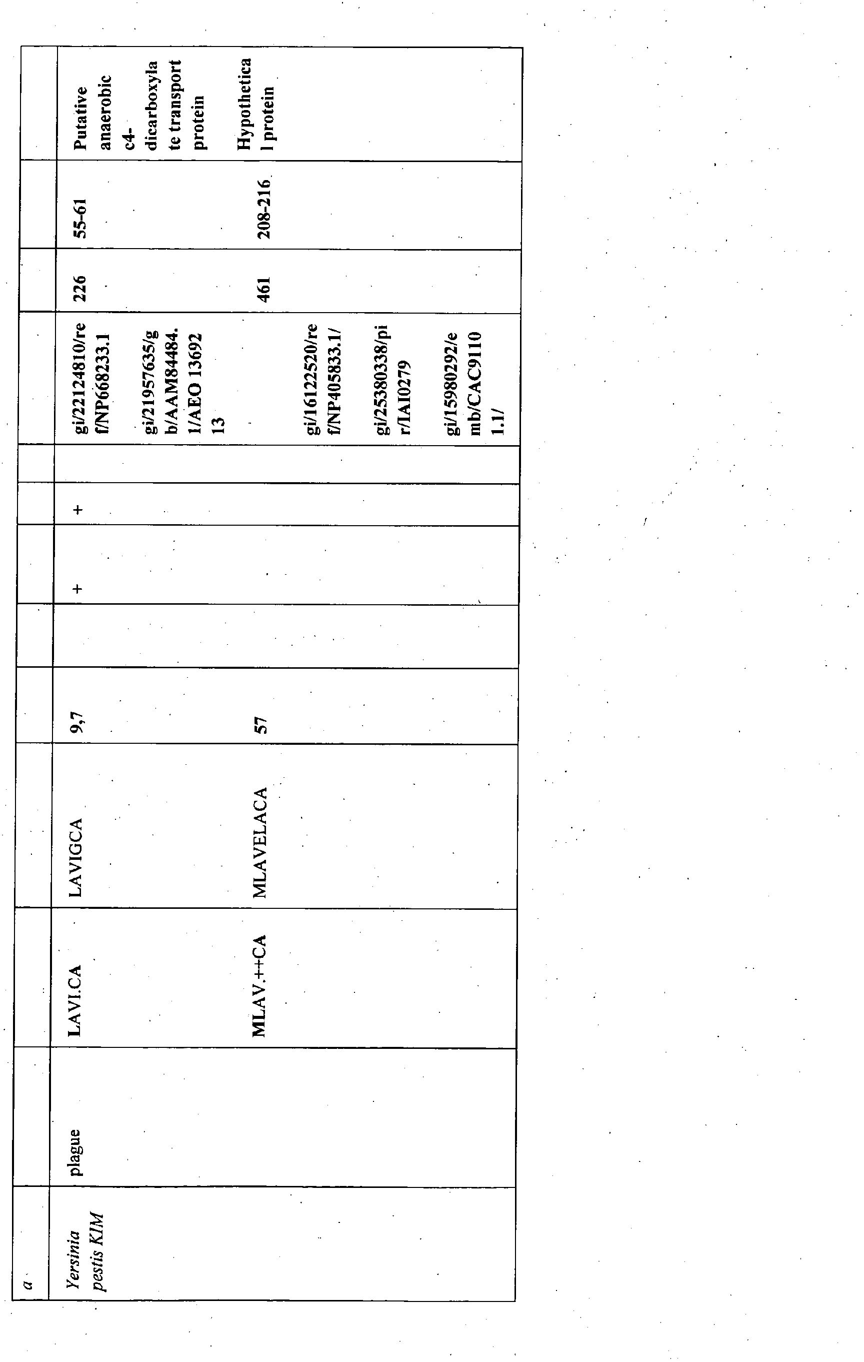

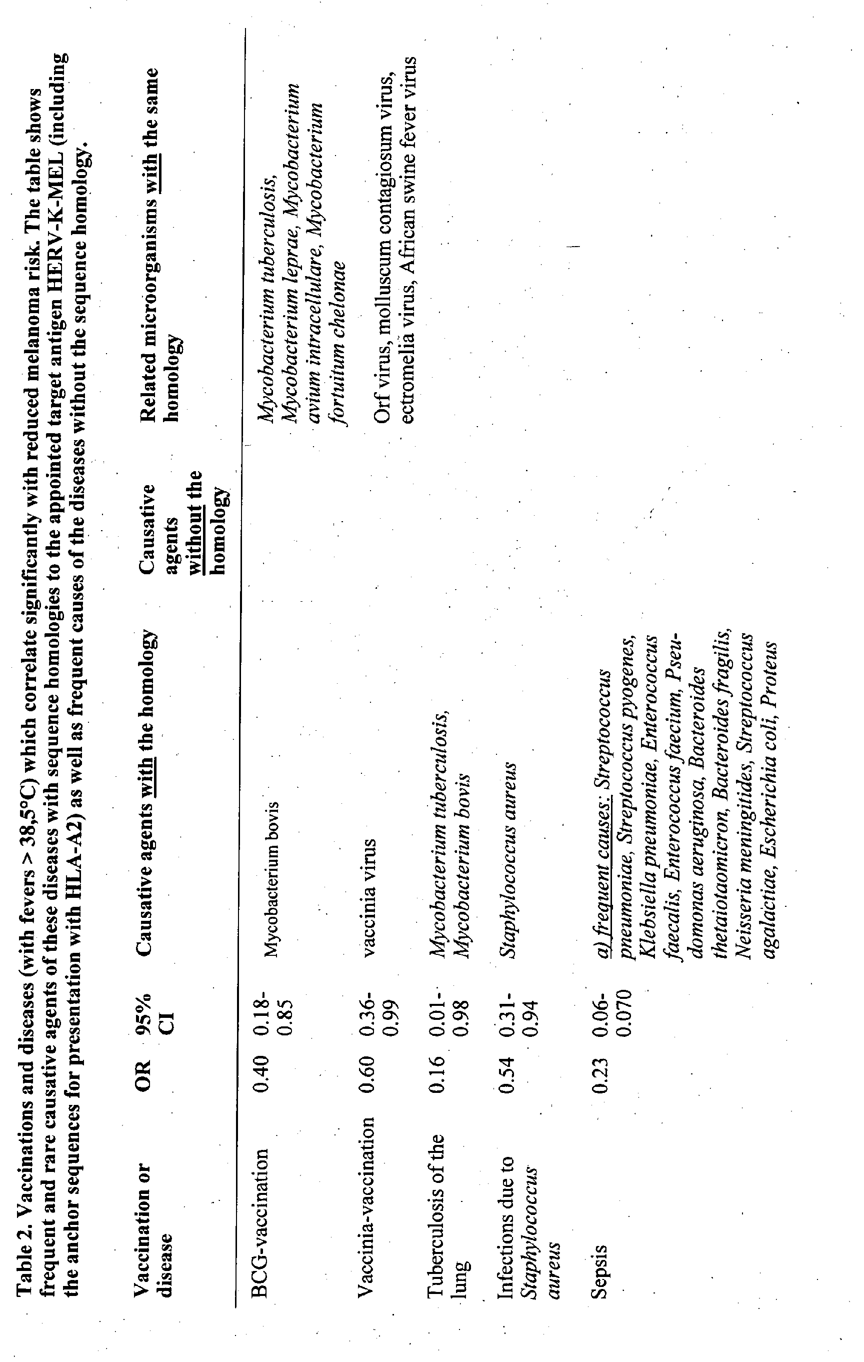

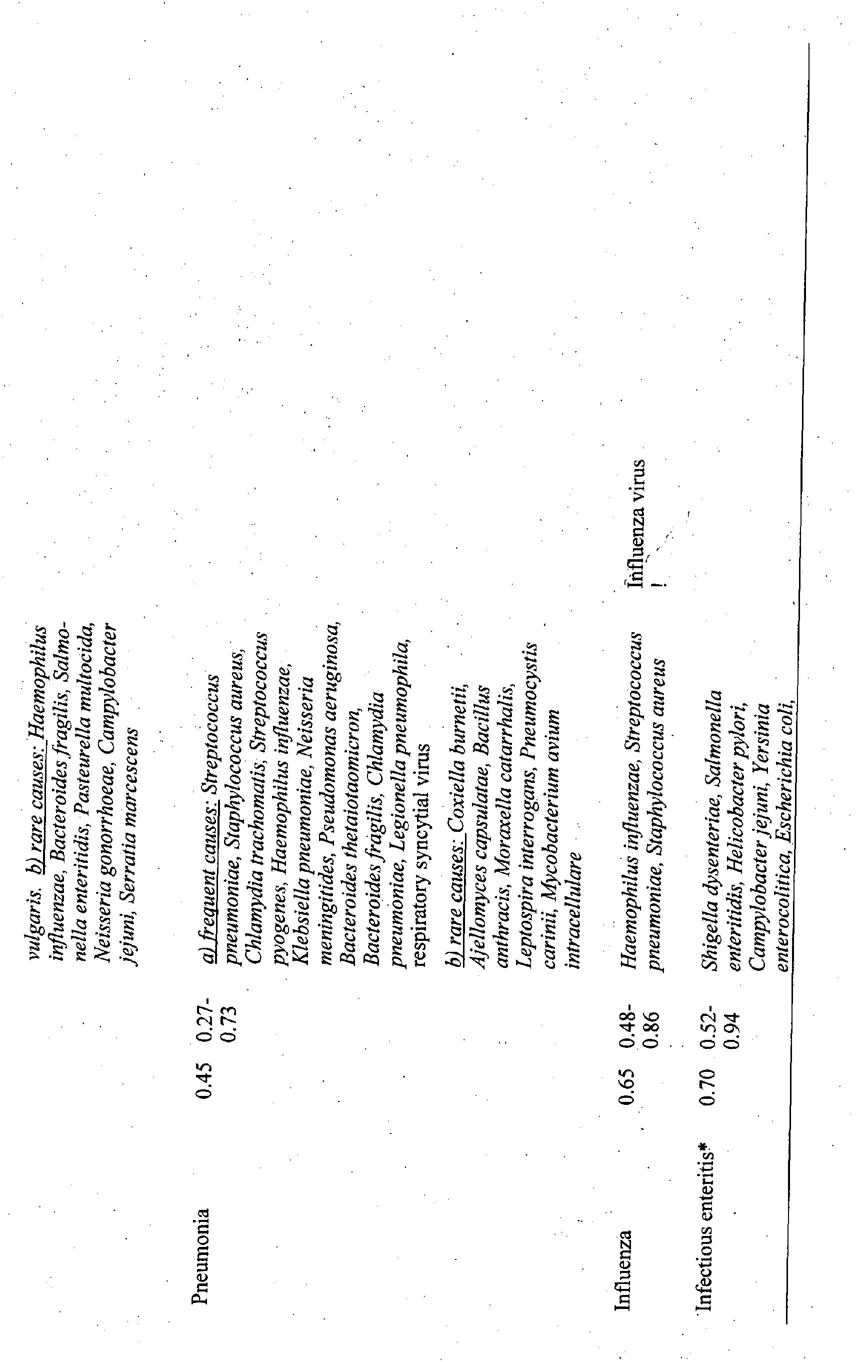

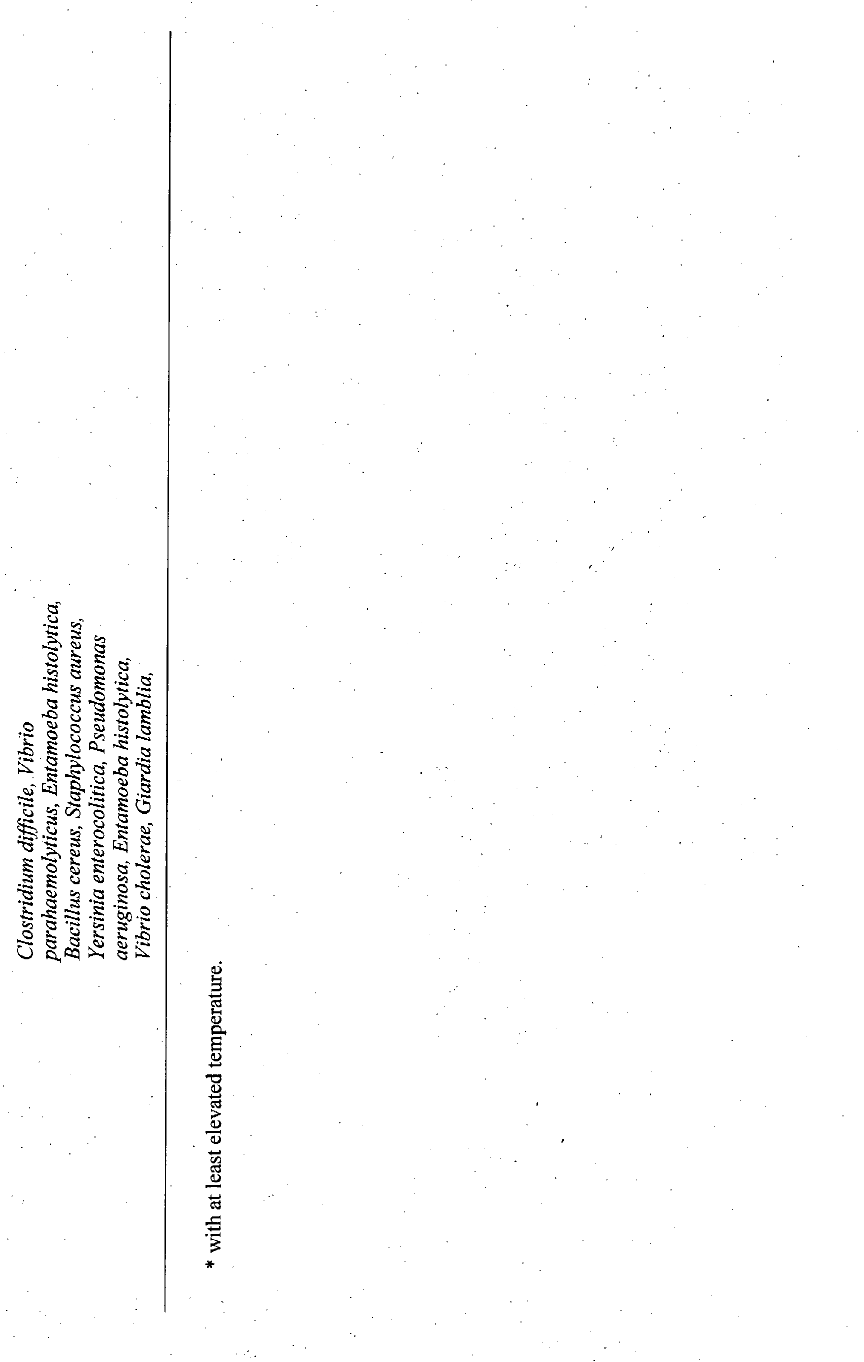

**Table 3.**

| Induction of an immune response against melanoma cells by vaccination of Rhesus monkeys against yellow fever. Pre-vaccinal and four weeks postvaccinal titers in an indirect immunofluorescence test are given using melanoma cells KII and Wörl, respectively. Bound IgG-antibodies were detected with FITC-labelled anti-monkey-IgG (Dako, Hamburg). | | | | |
|---|---|---|---|---|
| | Melanoma cells | | | |
| Monkey # | KII | KII | Wörl | Wörl |
| | pre-vaccinal | post-vaccinal | pre-vaccinal | post-vaccinal |
| 1 | < 10 | 160 | < 10 | 320 |
| 2 | < 10 | 320 | < 10 | 320 |
| 3 | < 10 | 80 | < 10 | 160 |
| 4 | < 10 | 20 | 10 | 40 |

**Table 4.**

| Search for sequence homologies in human pathogenic micro-organisms with permutations of the HERV-K-MEL sequence by protein blast for short nearly exact matches. Only homologies with E < -100 were accepted. If more than three human pathogenic microorganisms had appeared, search would have been extended to look for distinct microorganisms. | | | |
|---|---|---|---|
| Number of trial | sequence | Number of organisms found | Found micro-organism |
| 1 | AMLCSIVAAV | 1 | Listeria monocytogenes |
| 2 | VAASLCMIAV | 0 | |
| 3 | ISAACVVAML | 1 | Mycobacterium leprae |
| 4 | CALVMAASVI | 1 | Enterococcus faecalis |
| 5 | CMVVVSILAA | 0 | |
| 6 | AMSCVIVVAL | 1 | Bordetella parapertussis |
| 7 | VMASVCILAV | 1 | Corynebacterium diphtheriae |
| 8 | VVISAIMCAV | 1 | Enterococcus faecalis |
| 9 | VILVACSMAV | 0 | |
| 10 | ASLAVMICVV | 0 | |

### Table 5. Pathogens which were identified to contain polypeptides homologous to the HERV-K MEL peptide and where vaccinations are available.

Live vaccines licensed: Japanese encephalitis B (PR of China), Mycobacterium bovis Bacille Calmette Guérin (BCG) (BCG vaccine, Organon Teknika Corporation, USA), Salmonella typhi (Typhoral L, Chiron Behring, Germany), Vaccinia virus (Smallpox vaccine, Wyeth Laboratories, Inc., USA), Vibrio cholerae (Orochol, Berna, Switzerland), yellow fever (Stamaril, Aventis Pasteur MSD, Germany)
Live vaccines not licensed in human medicine: Brucella mellitensis (licensed for use in veterinary medicine only), Venezuelan encephalitis virus (Investigatioal New Drug Product developed in U.S. Army Medical Unit, Fort Detrick, USA)
Inactivated vaccines licensed: Bacillus anthracis (Anthrax vaccine, Michigan Biologic Products, USA), Bordetella pertussis (Pac Mérieux, Aventis Pasteur MSD, Germany), Clostridium diphtheriae (Diphtherie-Adsorbat-Impfstoff, Chiron Behring, Germany, Clostridium tetani (Tetanol pur, Chiron Behring, Germany), Coxiella burnetii, Haemophilus influenzae (PedvaxHIB Liquid, Chiron Behring, Germany), Japanese encephalitis B virus (JE-VAX, Research Foundation for Microbial Diseases of Osaka University, USA), Mycobacterium fortuitum chelonae (Aningzochin, Germany), Neisseria meningitides (Mencevax ACWY, GlaxoSmithKline, Germany), Salmonella typhi (TYPHiM Vi, Aventis Pasteur MSD, Germany), Streptococcus pneumoniae (Pneumopur, Chiron Behring, Germany), tick borne encephalitis virus (Encepur, FSME-Vaccine Behring, Germany), Vibrio cholerae (Cholera Impfstoff, Behring, Germany), yersinia pestis (Plague vaccine, Michigan Biologic Products, USA)
Inactivated vaccines not licensed: Borrelia burgdorferi, Respiratory syncytial virus, Serratia marcescens (Coley's toxin)

### References

1. Bonanni P. Demographic impact of vaccination: a review. Vaccine 1999; 17 suppl. 3: 120-5
2. Kristensen I, Aaby P, Jensen H. Routine vaccinations and child survival: follow-up study in Guinea-Bissau, West Africa. Br Med J 2000; 321: 1435-9
3. Czarnetzki BM, Macher E, Suciu S et al. Long term adjuvant immunotherapy in stage I high risk malignant melanoma, comparing two BCG preparations versus non-treatment in a randomized multicenter study (EORTC protocol 18781). Eur J Cancer 1993; 29A: 1237-42.
4. Boyd LA. Intravesical Bacillus Calmette-Guerin for treating bladder cancer. Urol Nurs. 2003; 23: 189-91, 199
5. Coley WB. The treatment of malignant tumors by repeated inoculations of erysipelas. Historical article of 1882, reprinted in Clin Orthop 1991; 262: 3-11
6. Schiavetti F, Thonnard J, Colau D et al. A human endogenous retroviral sequence encoding antigen recognized on melanoma by cytolytic T lymphocytes. Cancer Res 2002; 62: 5510-6
7. Grange JM, Stanford JL. BCG vaccination and cancer. Tubercle 1990; 71: 61-4
8. Abel U, Becker N, Angerer R et al. Common infections in the history of cancer patients and controls. J Cancer Res Clin Oncol 1991; 117, 339-44
9. Pfahlberg A, Kölmel KF, Grange JM et al. Inverse association between melanoma and previous vaccinations against tuberculosis and smallpox: results of the FEBIM study. J Invest Dermatol 2002; 119: 570-5
10. Krone B, Kölmel KF, Grange JM et al. Impact of vaccinations and infectious diseases on melanoma risk - evaluation of an EORTC case-control-study. Eur J Cancer 2003; 119: 570-5
11. Koelmel KF, Pfahlberg A, Mastrangelo G et al. Infections and melanoma risk: results of a multicentre EORTC case-control study. Melanoma Res 1999; 9: 511-9
12. KF Koelmel KF, Krone B, Grange JM et al. Influence of immunisation with vaccinia or BCG and various infectious diseases preceding the removal of the primary tumor on prognosis of malignant melanoma patients. An EORTC cohort study on 574 patients. Submitted for publication
13. Mumberg D, Muller R, Funk M. Regulatable promoters of Saccharomyces cerevisiae: comparison of transcriptional activity and their use for heterologous expression. Nucl Acids Res 1994, 22: 5767-8
14. Boorsma M, Hoenke S, Marrero A, et al. Bioprocess applications of a Sindbis virus-based temperature-inducible expression system. Biotechnol Bioeng 2002; 79: 602-9
15. Schlesinger S. Alphaviruses-vectors for the expression of heterologous genes. Trends Biotechnol 1993; 11: 18-22
16. He TC, Zhou S, da Costa LT, Yu J, Kinzler KW, Vogelstein B. A simplified system for generating recombinant adenoviruses. Proc Natl Acad Sci USA 1998; 95: 2509-14
17. Osei-Kwasi M, Dunyo SK, Koram KA et al. Antibody response to 17D yellow fever vaccine in Ghanaian infants. Bull World Health Organ 2001; 79: 1056-9
18. Lowrie DB. DNA vaccination: an update. Methods Mol Med. 2003; 87: 377-90
19. Gurunathan S, Klinman DM, Seder RA. DNA vaccines: Immunology, application, and optimization.Annu Rev Immuol 2000; 18: 927-1025
20. Haupt K, Roggendorf M, Mann K. The potential of DNA vaccination against tumor-associated antigens for antitumor therapy. Experimental Biology and Medicine 2002; 227: 227-37
21. D'Souza, Rosseels V, Denis O et al. Improved tuberculosis DNA vaccines by formulation in cationic lipids. Infection and Immunity 2002; 70: 3681-8
22. Condon C, Watkins SC, Celuzzi CM et al. DNA-based immunization by *in vivo* transfection of dendritic cells. Nature Medicine 1996; 2: 1122-8
23. Kent SJ, Stallard V, Corey L et al. Analysis of cytotoxic T-lymphocyte responses to SIV proteins in SIV-infected macaques using antigen-specific stimulation with recombinant vaccinia and fowlpox viruses. AIDS Res Hum Retroviruses 1994; 10:551-60
24. Fries LF, Tartaglia J, Taylor J et al. Human safety and immunogenicity of a canarypoxrabies glycoprotein recombinant vaccine: an alternative poxvirus vector system. Vaccine 1996; 14: 428-34.
25. McConkey SJ, Reece WH, Moorthy VS et al. Enhanced T-cell immunogenicity of plasmid DNA vaccines boosted by recombinant modified vaccinia virus Ankara in humans. Nature Medicine 2003; 9: 729-735.
26. Niedrig M, Stolte N, Fuchs D, Hunsmann G, Stahl-Hennig C. Intra-nasal infection of macaques with yellow Fever (YF) vaccine strain 17D: a novel and economical approach for YF vaccination in man. Vaccine 1999; 17: 1206-10.
27. Blanchard A, Montagnier L. AIDS-associated mycoplasmas. Annu Rev Microbiol 1994; 48: 687-712.

SEQUENCE LISTING

<110>  Georg-August-Universität Göttingen

<120>  Vaccination against malignant melanoma

<130>  K62381EP

<160>  98

<170>  PatentIn version 3.1

<210>  1
<211>  9
<212>  PRT
<213>  homo sapiens

<400>  1

Met Leu Ala Val Ile Ser Cys Ala Val
1               5

<210>  2
<211>  10
<212>  PRT
<213>  homo sapiens

<400>  2

Ala Met Leu Ala Val Ile Ser Cys Ala Val
1               5                   10

<210>  3
<211>  8
<212>  PRT
<213>  Ajellomyces capsulatus

<400>  3

Seite 1

Leu Ala Val Met Cys Lys Ala Val
1               5

<210>  4

<211>  9

<212>  PRT

<213>  Babesia bovis


<400>  4

Ala Ala Leu Ala Leu Gly Ser Cys Ala
1               5

<210>  5

<211>  9

<212>  PRT

<213>  Bacillus anthracis


<400>  5

Met Leu Gly Ala Val Ile Ser Ala Val
1               5

<210>  6

<211>  9

<212>  PRT

<213>  Bacillus cereus


<400>  6

Met Leu Gly Ala Val Ile Ser Ala Val
1               5

<210>  7

<211>  8

<212>  PRT

<213>  Bacteroides thetaiotaomicron


<400>  7

Met Leu Val Leu Leu Ser Cys Ala
1               5

Seite 2

<210>  8

<211>  10

<212>  PRT

<213>  Bacteroides fragilis


<400>  8

Met Leu Ala Val Pro Leu Leu Ala Cys Ala
1               5                   10


<210>  9

<211>  7

<212>  PRT

<213>  Bartonella bacilliformis


<400>  9

Leu Thr Val Val Glu Cys Ala
1               5


<210>  10

<211>  12

<212>  PRT

<213>  Bordetella pertussis


<400>  10

Ala Met Val Ala Val Phe Ala Cys Phe Leu Ala Val
1               5                   10


<210>  11

<211>  7

<212>  PRT

<213>  Bordetella pertussis


<400>  11

Leu Ala Gly Ile Pro Cys Ala
1               5


<210>  12

<211>  7

Seite 3

<212>  PRT
<213>  Borrelia burgdorferi

<400>  12
Leu Ala Leu Ile Gly Cys Ala
1               5

<210>  13
<211>  9
<212>  PRT
<213>  Brucella mellitensis, B. abortus, B. suis

<400>  13
Ala Met Leu Ala Ile Ala Gly Cys Ala
1               5

<210>  14
<211>  8
<212>  PRT
<213>  Brucella mellitensis, B. abortus, B. suis

<400>  14
Met Leu Leu Val Val Ala Cys Ala
1               5

<210>  15
<211>  7
<212>  PRT
<213>  Campylobacter jejuni

<400>  15
Leu Leu Ala Val Ile Ala Val
1               5

<210>  16
<211>  11
<212>  PRT
<213>  Chlamydia trachomatis

Seite 4

<400>  16

Ala Met Phe Val Ala Ile Val Ser Cys Ala Val
1               5                   10

<210>  17

<211>  9

<212>  PRT

<213>  Chlamydia trachomatis

<400>  17

Met Met Asp Ala Ile Ser Cys Ala Val
1               5

<210>  18

<211>  11

<212>  PRT

<213>  Clostridium tetani

<400>  18

Ala Met Ile Ala Val Val Leu Ser Cys Ala Ile
1               5                   10

<210>  19

<211>  9

<212>  PRT

<213>  Corynebacterium diphtheriae

<400>  19

Leu Leu Ala Leu Val Leu Val Ala Val
1               5

<210>  20

<211>  7

<212>  PRT

<213>  Coxiella burnetii

<400>  20

Seite 5

Leu Ala Ile Val Cys Cys Ala
1               5

<210>  21

<211>  9

<212>  PRT

<213>  Entamoeba histolytica


<400>  21

Ala Met Leu Val Val Ser Ser Cys Ala
1               5

<210>  22

<211>  9

<212>  PRT

<213>  Enterococcus faecalis


<400>  22

Ala Thr Leu Asn Ile Ile Ser Cys Ala
1               5

<210>  23

<211>  9

<212>  PRT

<213>  Enterococcus faecium


<400>  23

Ala Val Leu Ala Val Ile Cys Ala Ile
1               5

<210>  24

<211>  10

<212>  PRT

<213>  Escherichia coli


<400>  24

Ala Met Leu Ala Val Val Ser Gly Ala Val
1               5                   10

Seite 6

<210>  25

<211>  9

<212>  PRT

<213>  Escherichia coli


<400>  25

Leu Leu Ala Ile Ala Ser Cys Ala Val
1               5

<210>  26

<211>  7

<212>  PRT

<213>  Fusobacterium nucleatum


<400>  26

Leu Gly Val Ile Thr Cys Ala
1               5

<210>  27

<211>  10

<212>  PRT

<213>  Giardia lamblia


<400>  27

Ala Val Ile Ala Val Ile Gly Cys Ala Val
1               5                   10

<210>  28

<211>  10

<212>  PRT

<213>  Giardia lamblia


<400>  28

Met Leu Pro Tyr Val Ile Ser Ser Ala Val
1               5                   10

<210>  29

<211>  9

Seite 7

<212>  PRT
<213>  Haemophilus ducreyi

<400>  29
Met Met Ala Leu Ile Ser Cys Leu Val
1               5

<210>  30
<211>  7
<212>  PRT
<213>  Haemophilus ducreyi

<400>  30
Leu Ala Val Val Thr Cys Ala
1               5

<210>  31
<211>  8
<212>  PRT
<213>  Haemophilus influenzae

<400>  31
Leu Ala Gly Val Ser Cys Asp Val
1               5

<210>  32
<211>  9
<212>  PRT
<213>  Helicobacter pylori

<400>  32
Met Leu Glu Val Val Asn Ser Ala Ile
1               5

<210>  33
<211>  10
<212>  PRT
<213>  Japanese encephalitis B virus

Seite 8

<400>  33

Ala Ser Leu Ala Val Val Ile Ala Cys Ala
1               5                   10

<210>  34

<211>  9

<212>  PRT

<213>  Klebsiella pneumoniae

<400>  34

Ala Phe Leu Ala Met Ile Pro Cys Ala
1               5

<210>  35

<211>  9

<212>  PRT

<213>  Klebsiella pneumoniae

<400>  35

Leu Leu Ile Val Ile Val Val Ala Val
1               5

<210>  36

<211>  10

<212>  PRT

<213>  Legionella pneumophila

<400>  36

Ala Val Leu Ala Leu Gly Ser Ser Ala Val
1               5                   10

<210>  37

<211>  9

<212>  PRT

<213>  Leishmania donovani

<400>  37

Seite 9

Ala Val Leu Ala Val Val Leu Cys Ala
1               5

<210>  38

<211>  13

<212>  PRT

<213>  Leishmania major


<400>  38

Ala Ile Leu Ala Val Ile Thr Tyr Ser Phe Cys Ser Val
1               5                   10

<210>  39

<211>  8

<212>  PRT

<213>  Leishmania major


<400>  39

Met Lys Ala Val Leu Ser Cys Ala
1               5

<210>  40

<211>  9

<212>  PRT

<213>  Leptospira interrogans


<400>  40

Met Leu Gly Phe Val Ser Cys Ile Val
1               5

<210>  41

<211>  6

<212>  PRT

<213>  Listeria innocua


<400>  41

Met Leu Ser Cys Ala Val
1               5

Seite 10

<210> 42

<211> 11

<212> PRT

<213> Listeria monocytogenes

<400> 42

Ala Met Leu Ala Ala Ile Asp Tyr Phe Cys Ala
1               5                   10

<210> 43

<211> 6

<212> PRT

<213> Listeria monocytogenes

<400> 43

Met Leu Ser Cys Ala Val
1               5

<210> 44

<211> 7

<212> PRT

<213> Moraxella catarrhalis

<400> 44

Leu Ala Val Ile Ala Ala Val
1               5

<210> 45

<211> 8

<212> PRT

<213> Molluscum contagiosum

<400> 45

Leu Ala Gly Ala Ala Cys Ala Val
1               5

<210> 46

<211> 11

Seite 11

<212>  PRT
<213>  Mycobacterium bovis

<400>  46
Leu Leu Ala Val Asp Val Val Pro Ile Ala Val
1               5                   10

<210>  47
<211>  7
<212>  PRT
<213>  Mycobacterium fortuitum-chelonae

<400>  47
Leu Leu Gly Val Val Ala Val
1               5

<210>  48
<211>  9
<212>  PRT
<213>  Mycobacterium leprae

<400>  48
Ala Met Leu Ala Ile Phe Ala Ala Val
1               5

<210>  49
<211>  9
<212>  PRT
<213>  Mycobacterium leprae

<400>  49
Met Ile Ala Leu Ile Ser Tyr Ala Val
1               5

<210>  50
<211>  10
<212>  PRT
<213>  Mycobacterium marinum

Seite 12

<400>  50

Ala Met Ser Ala Val Ala Ala Leu Ala Val
1               5                   10

<210>  51

<211>  9

<212>  PRT

<213>  Mycobacterium tuberculosis


<400>  51

Met Ile Ala Leu Ile Ser Tyr Ala Val
1               5

<210>  52

<211>  10

<212>  PRT

<213>  Mycoplasma penetrans


<400>  52

Ala Thr Leu Ala Val Ile Ala Ser Thr Val
1               5                   10

<210>  53

<211>  8

<212>  PRT

<213>  Neisseria gonorrhoes


<400>  53

Leu Ala Gly Val Ser Tyr Ala Val
1               5

<210>  54

<211>  8

<212>  PRT

<213>  Neisseria meningitides


<400>  54

Seite 13

Met Leu Gly Gly Ile Thr Cys Ala
1               5

<210>  55

<211>  9

<212>  PRT

<213>  Orf virus

<400>  55

Leu Leu Ala Val Ala Ala Val Ala Val
1               5

<210>  56

<211>  7

<212>  PRT

<213>  Pasteurella multocida

<400>  56

Leu Ala Val Val Thr Cys Ala
1               5

<210>  57

<211>  8

<212>  PRT

<213>  Pasteurella multocida

<400>  57

Leu Ala Gly Ile Ser Cys Asp Val
1               5

<210>  58

<211>  9

<212>  PRT

<213>  Pasteurella multocida

<400>  58

Met Leu Thr Ala Leu Gly Cys Ala Val
1               5

Seite 14

<210>  59

<211>  13

<212>  PRT

<213>  Plasmodium falciparum

<400>  59

Met Leu Tyr Tyr Gly Ser Tyr Ala Val Leu Ser Cys Ala
1               5                   10

<210>  60

<211>  10

<212>  PRT

<213>  Pneumocystis carinii

<400>  60

Ala Val Leu Ala Thr Ile Ser His Ala Ile
1               5                   10

<210>  61

<211>  9

<212>  PRT

<213>  Pseudomonas aeruginosa

<400>  61

Ala Met Leu Val Ile Ile Ser Ala Val
1               5

<210>  62

<211>  10

<212>  PRT

<213>  Pseudomonas aeruginosa

<400>  62

Ala Met Pro Glu Val Ile Ser Val Asn Val
1               5                   10

<210>  63

<211>  9

Seite 15

<212>  PRT
<213>  Respiratory syncytial virus

<400>  63
Ala Met Asp Ala Val Lys Ile Asn Cys
1               5

<210>  64
<211>  8
<212>  PRT
<213>  Rickettsia rickettsii

<400>  64
Met Leu Ala Ile Ile Ser Ser Ala
1               5

<210>  65
<211>  8
<212>  PRT
<213>  Rickettsia typhi

<400>  65
Leu Asn Val Val Thr Thr Ala Val
1               5

<210>  66
<211>  9
<212>  PRT
<213>  Salmonella typhi

<400>  66
Met Leu Ala Met Ile Val Ser Ala Val
1               5

<210>  67
<211>  8
<212>  PRT
<213>  Salmonella typhi

Seite 16

<400> 67

Leu Ala Val Val Phe Ile Ala Val
1               5

<210> 68

<211> 9

<212> PRT

<213> Salmonella enterica

<400> 68

Met Leu Ala Met Ile Val Ser Ala Val
1               5

<210> 69

<211> 10

<212> PRT

<213> Serratia marcescens

<400> 69

Ala Phe Leu Ala Val Val His Cys Ala Val
1               5                   10

<210> 70

<211> 11

<212> PRT

<213> Shigella flexneri

<400> 70

Ala Met Leu Ala Leu Val Val Ser Gly Ala Val
1               5                   10

<210> 71

<211> 9

<212> PRT

<213> Staphylococcus aureus

<400> 71

Seite 17

Met Leu Ala Gly Ile Ser Val Ala Val
1               5

<210>  72

<211>  11

<212>  PRT

<213>  Streptococcus agalactiae


<400>  72

Ala Leu Leu Arg Ala Phe Ile Ser Cys Ala Val
1               5                   10

<210>  73

<211>  12

<212>  PRT

<213>  Streptococcus pneumoniae


<400>  73

Leu Leu Ala Val Val Thr Ile Val Phe Cys Ala Val
1               5                   10

<210>  74

<211>  10

<212>  PRT

<213>  Streptococcus pyogenes


<400>  74

Met Leu Lys Gln Ala Val Ser Cys Ala Ile
1               5                   10

<210>  75

<211>  10

<212>  PRT

<213>  Tick-borne encephalitis virus


<400>  75

Leu Ala Val Val Leu Cys Leu Met Ala Val
1               5                   10

Seite 18

<210>  76

<211>  13

<212>  PRT

<213>  Toxoplasma gondii


<400>  76

Ala Met Leu Ala Leu Gly Asp Lys Ile Gly Ser Ala Val
1               5                   10


<210>  77

<211>  9

<212>  PRT

<213>  Toxoplasma gondii


<400>  77

Met Gln Leu Ala Cys Ala Ser Cys Ala
1               5


<210>  78

<211>  9

<212>  PRT

<213>  Treponema pallidum


<400>  78

Ala Met Leu Ala Val Leu Ser Phe Ala
1               5


<210>  79

<211>  7

<212>  PRT

<213>  Treponema pallidum


<400>  79

Met Leu Ala Val Arg Cys Ala
1               5


<210>  80

<211>  9

Seite 19

<212>  PRT
<213>  Treponema pallidum

<400>  80
Ala Leu Leu Ser Leu Ser Cys Ala Val
1               5

<210>  81
<211>  9
<212>  PRT
<213>  Trichomonas vaginalis

<400>  81
Met Leu Thr Ser Val Ser Tyr Pro Val
1               5

<210>  82
<211>  10
<212>  PRT
<213>  Tropheryma whippeli

<400>  82
Met Val Ala Leu Thr Ile Ser Cys Ala Ile
1               5                   10

<210>  83
<211>  7
<212>  PRT
<213>  Trypanosoma brucei

<400>  83
Leu Ala Ile Ile Ser Ser Ala
1               5

<210>  84
<211>  11
<212>  PRT
<213>  Trypanosoma gambiense

Seite 20

<400>  84

Ala Met Asp Leu Lys Ile Val Ser Ala Ala Val
1               5                   10

<210>  85

<211>  7

<212>  PRT

<213>  Trypanosoma cruzi

<400>  85

Leu Ala Ile Ile Ser Ser Ala
1               5

<210>  86

<211>  10

<212>  PRT

<213>  Vaccinia virus

<400>  86

Ala Ser Leu Ala Val Val Ile Ala Cys Ala
1               5                   10

<210>  87

<211>  7

<212>  PRT

<213>  Venezualian equine encephalitis virus

<400>  87

Leu Ala Val Leu Cys Cys Ala
1               5

<210>  88

<211>  7

<212>  PRT

<213>  Vibrio cholerae

<400>  88

Seite 21

Leu Ala Val Val Thr Cys Ala
1               5

<210>  89

<211>  9

<212>  PRT

<213>  Vibrio parahaemolyticus


<400>  89

Met Leu Ala Ala Ile Ala Val Ala Val
1               5

<210>  90

<211>  10

<212>  PRT

<213>  Vibrio parahaemolyticus


<400>  90

Ala Met Leu Ala Ala Ile Met Cys Ile Val
1               5                   10

<210>  91

<211>  9

<212>  PRT

<213>  Vibrio vulnificus


<400>  91

Met Leu Ala Leu Ile Thr Ile Ala Val
1               5

<210>  92

<211>  7

<212>  PRT

<213>  Vibrio vulnificus


<400>  92

Leu Ser Val Ile Ala Cys Ala
1               5

Seite 22

<210> 93

<211> 7

<212> PRT

<213> Vibrio vulnificus


<400> 93

Leu Ala Val Val Thr Cys Ala
1               5


<210> 94

<211> 7

<212> PRT

<213> Yellow fever virus


<400> 94

Leu Ala Val Ser Ser Ala Val
1               5


<210> 95

<211> 9

<212> PRT

<213> Yersinia enterocolitica


<400> 95

Leu Asn Asn Val Val Ala Cys Ala Val
1               5


<210> 96

<211> 7

<212> PRT

<213> Yersinia pestis KIM


<400> 96

Leu Ala Val Ile Gly Cys Ala
1               5


<210> 97

<211> 9

Seite 23

<212>  PRT
<213>  Yersinia pestis KIM

<400>  97
Met Leu Ala Val Glu Leu Ala Cys Ala
1               5

<210>  98
<211>  6
<212>  PRT
<213>  Proteus vulgaris

<400>  98
Leu Ala Val Ser Cys Ala
1               5

Seite 24

## Claims

1. Use of a first peptide with defined homology to SEQ ID NO:1 (HERV K-MEL) and/or a composition comprising said first peptide sequence for the preparation of a vaccine for the prevention of a disease, wherein one of the causative agents of said disease comprises a polypeptide comprising a second peptide sequence with defined homology to SEQ ID NO:1 (HERV K-MEL) which second peptide sequence is not identical with said first peptide sequence, wherein the vaccine is not a vaccinia vaccine or the BCG vaccine.

2. Use of claim 1, wherein the disease is a malignancy, preferably wherein said malignancy is selected from the group consisting of melanoma, carcinoma of bladder, carcinoma of kidney, carcinoma of esophagus, sarcoma, lymphoma and leukemia, more preferably wherein said malignancy is melanoma.

3. Use of claim 1, wherein the disease is an infectious disease, preferably wherein said infectious disease is selected from the group consisting of acute respiratory disease, African sleeping disease, anthrax, arthritis, babesiosis, chagas disease, chancroid, cholera, dell wart, diphtheria, duodenal ulcera, ecthyma contagiosum, endemic typhus, enteritis, epidemic typhus, erysipelas, gastritis, gonorrhea, histoplasmosis, intra-abdominal and pelvic infections, Japanese encephalitis B, kala-azar, leprosy, listeriosis, local invasive infection, lyme disease, lymphogranuloma inguinale, malaria tropica, meningitis, morbus Bang, morbus Weil, Oroya fever, peritonitis, pertussis, plague, pneumonia, Q fever, rocky mountain spotted fever, sepsis, sinusitis, smallpox, swimming pool granuloma, syphilis, tetanus, tick-borne encephalitis, toxoplasmosis, trachoma, trichomoniasis, tuberculosis, ulcera, urinary tract infection, Venezuelan equine encephalitis, wound infection, and yellow fever.
more preferably wherein said disease is selected from the group consisting of acute respiratory disease, anthrax, arthritis, chancroid, enteritis, histoplasmosis, intra-abdominal and pelvic infections, leprosy, local invasive infection, lyme disease, lymphogranuloma inguinale, morbus Bang, peritonitis, sinusitis, toxoplasmosis, and trichomoniasis.
most preferably wherein said disease is selected from the group consisting of African sleeping disease, chagas disease, cholera, gonorrhea, kala-azar, listeriosis, malaria tropica, meningitis, morbus Weil, pneumonia, sepsis, syphilis, trachoma, tuberculosis, urinary tract infection, and wound infection.

4. Use of any one of claims 1 to 3, particularly use of claim 2, wherein said first peptide with defined homology and/or said second peptide with defined homology each have at least 5, more preferably 6 and most preferably 7, amino acid residues in common with SEQ ID NO.1 when aligned with SEQ ID NO.1 to maximize the number of identical residues in such a way that only at position 5 and position 6 an insertion or deletion of one amino acid residue is allowed.

5. Use of any one of claims 1 to 3, wherein said first peptide with defined homology and/or said second peptide with defined homology each are derived from the peptide sequence MLAVISCAV or MLAVVSCAV in such a way that
a) either residue M at position 1 and residue A at position 8 remain unchanged, or that
b) residue L at position 2 and residue V at position 9 remain unchanged,
and that one, two or three, more preferably one or two, even more preferably one, of the other amino acid residues are exchanged for another amino acid residue, preferably wherein the amino acid exchange is a semi-conservative exchange, more preferably wherein the amino acid exchange is a conservative exchange,
and/or wherein one or two amino acid residues can be inserted or deleted at any position between
amino acid residue 1 and 8 in the case of alternative a) or
amino acid residue 2 and 9 in the case of alternative b),
wherein preferably said insertion is an insertion of one or two, more preferably one, amino acid residues at position 5 or 6, or
wherein preferably said deletion is a deletion of one or two, more preferably one, amino acid residues at position 5 or 6.

6. Use of any one of claims 1 to 3, particularly use of claim 2, wherein the first and/or second peptide is selected from the group consisting of SEQ ID NO:1-97.

7. Use of any one of the preceding claims, wherein said first peptide with defined homology to SEQ ID NO:1 (HERV K-MEL) and/or said composition comprising said first peptide sequence is comprised in a vaccine and said vaccine is selected from the group consisting of the yellow fever vaccine, the Japanese encephalitis B vaccine, the Venezuelan equine encephalitis vaccine, the typhoid fever vaccine, the cholera vaccine, the rickettsia typhi vaccine, the pneumococci vaccine, the Friedmann vaccine (Mycobacterium chelonae), the vaccine against respiratory syncytial virus, the vaccine against pertussis, the vaccine against tick borne encephalitis, the Coley's toxin vaccine, the vaccine against Q-fever, the vaccine against brucella, the vaccine against anthrax, the vaccine against plague and the modified vaccinia vaccine Ankara (MVA), preferably selected from the group consisting of the yellow fever vaccine, the Japanese encephalitis B vaccine, the Venezuelan equine encephalitis vaccine, the typhoid fever vaccine, the cholera vaccine and the rickettsia typhi vaccine, and most preferably the yellow fever vaccine.

8. Use any one of the preceding claims, wherein the vaccine is to be administered to a human subject above age 10, wherein the human subject has previously been vaccinated as a child with a vaccine according to claims 23 or 24 or with smallpox- or BCG-vaccine.

9. Use of any one of claims 1-7, wherein the vaccine is to be administered to a child below age 2 years, preferably to a baby below age 1 year.

10. Use of claim 9, wherein the vaccine has further been adapted so as to be administrable to a child below age 2 years, preferably to a baby below age 1 year.

11. Method of vaccinating a subject for the prophylaxis of a disease as specified in any one of claims 1 to 3, preferably wherein the disease is a malignancy, more preferably
wherein the malignancy is melanoma, wherein the method comprises the step of administering a first peptide as defined in claim 1, preferably as defined in claim 4 or alternatively as defined in claim 5, and/or a composition comprising said peptide sequence to a subj ect.

12. Method of claim 11, wherein the subject is a human subject.

13. Method of claims 11 or 12, wherein said peptide is administered to a child below age 2 years, preferably to a baby below age 1 year.

14. Peptide as defined in claims 4 and/or 5 for use in vaccination for the prevention of a malignancy, wherein the peptide does not have the peptide sequence SEQ ID NO:1.

15. Peptide of claim 14, wherein the peptide is selected from the group consisting of SEQ ID NO:3-97.

16. Peptide of any one of claims 14 to 15, wherein the peptide has a length of from 8 to 12 amino acid residues.

17. Peptide of any one of claims 14 to 16, wherein the peptide is further coupled to a carrier polypeptide, preferably to a polypeptide derived from tetanus toxoid or choleratoxin.

18. Peptide of any one of claims 14 to 17, wherein the peptide is capable of being presented by the HLA A2 or another frequently occurring HLA class I molecule.

19. Peptide of any one of claims 14 to 18, wherein the peptide comprises an anchor sequence for presentation by HLA A2 class I.

20. Polypeptide comprising a peptide sequence as defined in claims 4 and/or 5 for use in vaccination for the prevention of a malignancy wherein said polypeptide is a fusion polypeptide which is not a naturally occurring polypeptide.

21. Polypeptide comprising a peptide sequence as defined in claims 4 and/or 5 for use in vaccination for the prevention of a malignancy wherein said polypeptide is selected from the group consisting of SEQ ID NO: 3-97, preferably wherein the polypeptide is derived from Mycobacterium bovis, Mycobacterium tuberculosis, vaccinia virus, Staphylococcus aureus, yellow fever virus, Chlamydia pneumoniae, Chlamydia trachomatis, Entamoeba histolytica, Giardia lamblia, Haemophilus ducreyi, Japanese encephalitis B, Klebsiella pneumoniae, Leishmania donovani, Moraxella catarrhalis, Mycobacterium leprae, Pasteurella multocida, Pseudomonas aeruginosa, Salmonella typhi, Serratia marcescens, Shigella flexneri, Treponema pallidum, Venezuelan equine encephalitis virus, Vibrio cholerae, Vibrio parahaemolyticus, Yersinia enterocolitica, Bartonella bacilliformis, Bordetella pertussis, Brucella mellitensis, Fusobacterium nucleatum, Haemophilus influenzae, Leishmania major, Listeria monocytogenes, Neisseria gonorrhoe, Neisseria meningitides, orf-virus, Pasteurella multocida, Pneumocystis carinii, Proteus vulgaris, Pseudomonas aeruginosa Rickettsia rickettsii, Sahnonella typhi, Salmonella enterica, Streptococcus agalactiae, Streptococcus pyogenes, Tick-borne-encephalitis virus, Trichomonas vaginalis, Toxoplasma gondii, Trypanosoma gambiense, Yersinia pestis, Ajellomyces capsulatus, Babesisa bovis, Bacillus anthracis, Bacteroides thetaiotaomicron, Bacteroides fragilis, campylobacter jejuni, Clostridium tetani, Corynebacterium diphtheriae, Enterococcus faecalis, Enterococcus faecium, Escherichia coli, Giardia lamblia, Helicobacter pylori, Legionella pneumophila, Leptospira interrogans, Listeria innocua, Molluscum contagiosum, Mycobacterium fortuitum-chelonae, Mycobacterium marinum, Mycobacterium leprae, Mycoplasma penetrans, Pasteurella multocida, Plasmodium falciparum, respiratory syncytial virus, Tropheryma whippeli, Trypanosoma brucei, Trypanosoma cruzi, Vibrio parahaemolyticus, Vibrio vulnificus, Yersinis pestis,
more preferably wherein the polypeptide is derived from Mycobacterium bovis, Mycobacterium tuberculosis, vaccinia virus, Staphylococcus aureus, Yellow fever virus, Chlamydia pneumoniae, Chlamydia trachomatis, Entamoeba histolytica, Giardia lamblia, Haemophilus ducreyi, Japanese encephalitis B, Klebsiella pneumoniae, Leishmania donovani, Moraxella catarrhalis, Mycobacterium leprae, Pasteurella multocida, Pseudomonas aeruginosa, Salmonella typhi, Serratia marcescens, Shigella flexneri, Treponema pallidum, Venezuelan equine encephalitis virus, Vibrio cholerae, Vibrio parahaemolyticus and Yersinia enterocolitica ,
most preferably wherein the polypeptide is derived from Mycobacterium bovis, Mycobacterium tuberculosis, vaccinia virus, Staphylococcus aureus and yellow fever virus

22. Composition comprising a peptide or polypeptide according to any one of claims 14-21, further comprising an adjuvant, for use in vaccination for the prevention of a malignancy.

23. Vaccine suitable for use in children, in particular adapted for use in infants and/or babies, comprising a peptide or polypeptide according to any one of claims 14-21.

24. Vaccine of claim 23 in unit dosage form, wherein the unit dosage is 10% - 90% of the unit dosage for adults, preferably 20% to 80%, more preferably 30% to 70% and most preferably 40% to 60%.

25. Vaccine of claim 23, comprising two or more different peptides according to any one of claims 14 to 19, two or more different polypeptides according to claims 20 or 21, or at least one peptide according to any one of claims 14 to 19 and at least one polypeptide according to claims 20 or 21.

26. DNA-based vaccine comprising an expression construct comprising a promoter functional in eukaryotic cells and a polynucleotide encoding at least one peptide according to any one of claims 14 to 19 and/or at least one polypeptide according to claims 20 or 21, wherein said polynucleotide is positioned sense to and under the control of said promoter, for use in vaccination for the prevention of a malignancy.

27. DNA-based vaccine of claim 26, wherein the polynucleotide encodes 5 to 30 copies, preferably 10 to 20 copies, of the peptide according to any one of claims 14 to 19 and/or 2 to 10 copies of the polypeptide according to claims 20 or 21.

28. DNA-based vaccine of claim 27, wherein the multiple copies of said peptide or said polypeptide are encoded in the context of a single fusion polypeptide.

29. DNA-based vaccine of any one of claims 26 to 28, further comprising a polypeptide of modified vacciniavirus Ankara (MVA), canary-pox-virus, adenovirus, sindbis-Virus, dengue virus, yellow fever virus, measles virus, sendai virus, polio virus, papilloma virus, rota virus, Venezuelan encephalitis virus, semliki forest virus and hepatitis A virus.

30. DNA-based vaccine of any one of claims 26 to 29, wherein the DNA-based vaccine is in the form of a viral vector, in particular in the form of a modified vacciniavirus Ankara (MVA), canary-pox-virus, adenovirus, sindbis-Virus, dengue virus, yellow fever virus, measles virus, sendai virus, polio virus, papilloma virus, rota virus, Venezuelan encephalitis virus, semliki forest virus and hepatitis A virus. Preferred are vectors of the pox group, papilloma, Venezuelan encephalitis virus, Semliki forest virus, adeno virus and hepatitis A virus.
